(19) Europäisches Patentamt
European Patent Office
Office européen des brevets

(11) Veröffentlichungsnummer: **0 473 024 A1**

(12) **EUROPÄISCHE PATENTANMELDUNG**

(21) Anmeldenummer: **91113731.3**

(22) Anmeldetag: **16.08.91**

(51) Int. Cl.[5]: **C07D 209/94**, C07D 403/06, C07D 209/80, C07D 209/86, A61K 31/405, A61K 31/41

(30) Priorität: **29.08.90 DE 4027278**

(43) Veröffentlichungstag der Anmeldung:
**04.03.92 Patentblatt 92/10**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Müller, Ulrich E., Dr.**
**Claudiusweg 5**
**W-5600 Wuppertal(DE)**
Erfinder: **Niewöhner, Ulrich, Dr.**
**Gartenstrasse 3**
**W-5632 Wermelskirchen 3(DE)**
Erfinder: **Perzborn, Elisabeth, Dr.**
**Am Tescher Busch 13**
**W-5600 Wuppertal 11(DE)**
Erfinder: **Bischoff, Erwin, Dr.**
**Pahlkestrasse 73**
**W-5600 Wuppertal(DE)**
Erfinder: **Dellweg, Hans-Georg, Dr.**
**In den Birken 46**
**W-5600 Wuppertal(DE)**

(54) **Heterocyclisch substituierte Cycloalkano[b]-indolsulfonamide.**

(57) Heterocyclisch substituierte Cycloalkano[b]-indolsulfonamide können durch Umsetzung von entsprechenden N-unsubstituierten Indolen mit Acrylnitril, anschließende Verseifung, Veresterung oder Amidierung, oder durch Umsetzung von heterocyclisch substituierten Hydrazinen mit Cycloalkanonen oder durch nachträgliches Einführen der heterocyclischen Gruppe in entsprechende Indolderivate hergestellt werden. Die heterocyclisch substituierten Cycloalkano[b]-indolsulfonamide können zur Behandlung von thromboembolischen Erkrankungen, Ischämien, Arteriosklerose, Asthma, Allergien sowie zur Prophylaxe von Myocardinfarkten eingesetzt werden.

EP 0 473 024 A1

Rank Xerox (UK) Business Services

Die Erfindung betrifft heterocyclisch substituierte Cycloalkano[b]-indolsulfonamide, Verfahren zu ihrer Herstellung und ihre Verwendung in Arzneimitteln.

Es ist bereits bekannt, daß Cycloalkano[b]dihydroindol- und -indolsulfonamide eine thrombozytenaggregationshemmende Wirkung aufweisen [vgl. DOS 36 31 824].

Es wurden nun heterocyclisch substituierte Cycloalkano[b]-indolsulfonamide der allgemeinen Formel (I)

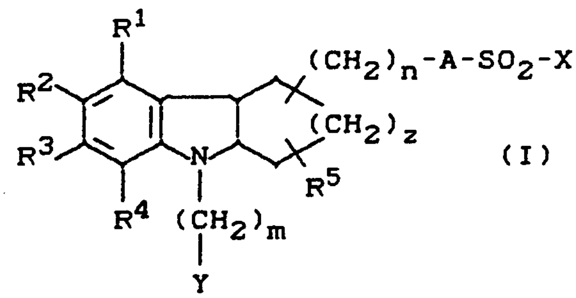

(I)

in welcher

$R^1$, $R^2$, $R^3$, $R^4$ und $R^5$ gleich oder verschieden sind und
- für Wasserstoff, Nitro, Cyano, Halogen, Trifluormethyl, Carboxy, Hydroxy oder Trifluormethoxy stehen, oder
- für eine Gruppe der Formel $-S(O)_wR^6$ stehen,
  worin
  $R^6$ - geradkettiges oder verzweigtes Alkyl mit bis zu 8 Kohlenstoffatomen oder Aryl mit 6 bis 10 Kohlenstoffatomen bedeutet, das seinerseits durch Halogen, Nitro, Cyano oder Trifluormethyl substituiert ist
  und
  w - eine Zahl 0, 1 oder 2 bedeutet,
  oder
- für geradkettiges oder verzweigtes Alkoxy oder Alkoxycarbonyl mit jeweils bis zu 10 Kohlenstoffatomen oder Benzyloxy stehen, oder
- für eine Gruppe der Formel $-NR^7R^8$ stehen,
  worin
  $R^7$ und $R^8$ gleich oder verschieden sind und
  - Wasserstoff, geradkettiges oder verzweigtes Alkyl oder Acyl mit jeweils bis zu 8 Kohlenstoffatomen oder
  - Aryl mit 6 bis 10 Kohlenstoffatomen bedeuten,
  oder
- für Cycloalkyl mit 3 bis 8 Kohlenstoffatomen oder
- für Aryl mit 6 bis 10 Kohlenstoffatomen stehen, oder
- für geradkettiges oder verzweigtes Alkyl oder Alkenyl mit jeweils bis zu 10 Kohlenstoffatomen stehen, die gegebenenfalls durch Halogen, Hydroxy, Carboxy, Cyano, Aryl mit 6 bis 10 Kohlenstoffatomen, geradkettiges oder verzweigtes Alkyl, Alkoxy oder Alkoxycarbonyl mit jeweils bis zu 8 Kohlenstoffatomen oder durch eine Gruppe der Formel
  $-S(O)_wR^6$ oder

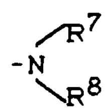

  substituiert sind,
  worin
  w, $R^6$, $R^7$ und $R^8$ die oben angegebene Bedeutung haben,
  oder
- für eine Gruppe der Formel

-D, -E-O-L-D, -E-NH-L-D, -E-CO-L-D oder -E-D
stehen,
worin
D - einen 3- bis 7-gliedrigen, gesättigten oder ungesättigten Heterocyclus mit bis zu 4 Heteroatomen aus der Reihe N, O oder S bedeutet, an den weitere aromatische oder heterocyclische Ringe ankondensiert sein können,
und
E und L gleich oder verschieden sind und
- eine direkte Bindung oder
- geradkettiges oder verzweigtes Alkylen oder Alkenylen mit bis zu 10 Kohlenstoffatomen bedeuten,

X

- für Aryl mit 6 bis 10 Kohlenstoffatomen steht, das gegebenenfalls bis zu 5-fach gleich oder verschieden durch Nitro, Halogen, Cyano, Trifluormethyl, Trifluormethoxy, Trifluormethylthio, Hydroxy, Carboxy, Aryl oder Aryloxy mit 6 bis 10 Kohlenstoffatomen, geradkettiges oder verzweigtes Alkoxy oder Alkoxycarbonyl mit jeweils bis zu 8 Kohlenstoffatomen oder durch geradkettiges oder verzweigtes Alkyl mit bis zu 8 Kohlenstoffatomen substituiert ist, wobei das Alkyl seinerseits durch Carboxy, Hydroxy, Alkoxycarbonyl mit bis zu 6 Kohlenstoffatomen, durch Aryl mit 6 bis 10 Kohlenstoffatomen,
oder
durch eine Gruppe der Formel $-S(O)_wR^6$ oder $-NR^7R^8$ substituiert sein kann,
worin
w, $R^6$, $R^7$ und $R^8$ die oben angegebene Bedeutung haben,
oder
- für geradkettiges oder verzweigtes Alkyl mit bis zu 8 Kohlenstoffatomen
oder
- für Trifluormethyl steht,

m - für die Zahl 1, 2, 3 oder 4 steht,
n - für die Zahl 0, 1 oder 2 steht,
z - für eine Zahl 1, 2, 3 oder 4 steht,
A - für eine direkte Bindung oder für eine Gruppe der Formel -NH- oder

-N-E-D
|

steht,
worin
D und E die oben angegebene Bedeutung haben,

Y

- für die Gruppe der Formel -CO-G steht,
worin
G - Hydroxy, Alkoxy mit bis zu 8 Kohlenstoffatomen, Phenoxy oder eine Gruppe der Formel $-NR^7R^8$ oder $-NH-SO_2-R^6$ bedeutet,
worin
$R^6$, $R^7$ und $R^8$ die oben angegebene Bedeutung haben,
oder
- für Tetrazolyl steht,

mit der Maßgabe, daß entweder der Substituent A für den Rest -N-E-D oder mindestens einer der Substituenten $R^1$, $R^2$, $R^3$, $R^4$ und $R^5$ für eine der Gruppen -D, -E-O-L-D, -E-NH-L-D, -E-CO-L-D oder -E-D stehen müssen,
gegebenenfalls in einer isomeren Form und ihre Salze, gefunden.

Die erfindungsgemäßen heterocyclisch substituierten Indolsulfonamide haben eines oder mehrere

asymmetrische Kohlenstoffatome und können daher in verschiedenen stereochemischen Formen existieren.

Die Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Außerdem können Regioisomere auftreten. Sowohl die einzelnen Isomeren als auch deren Mischungen sind Gegenstand der Erfindung.

Im folgenden werden beispielhaft isomere Formen der heterocyclisch substituierten Indolsulfonamide aufgeführt:

a) Cycloalkano[b]indolsulfonamide

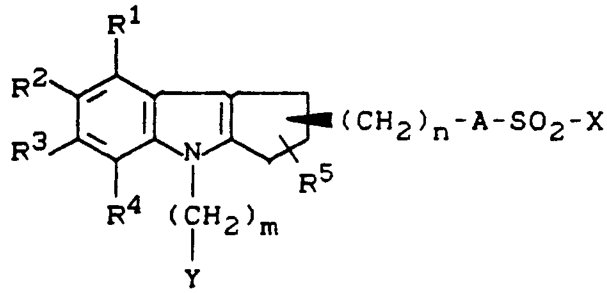

(II)

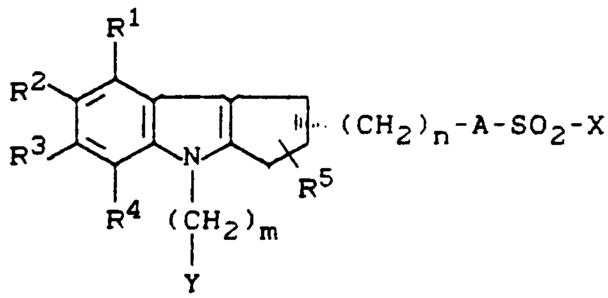

(III)

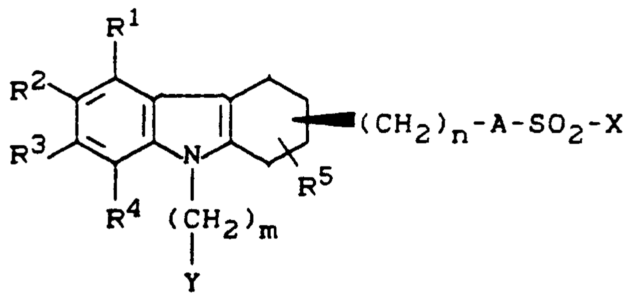

(IV)

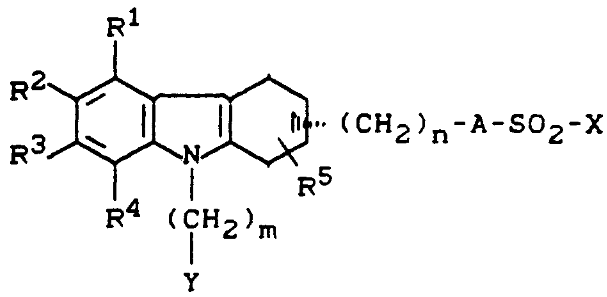

(V)

b) Cycloalkano[b]dihydro-indolsulfonamide

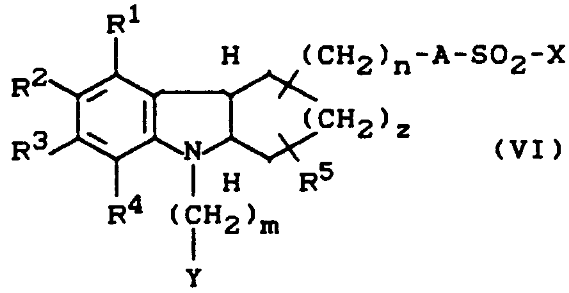

(VI)

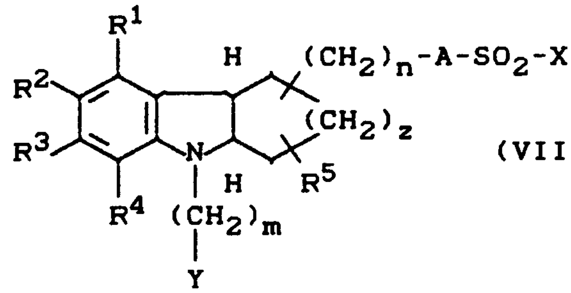

(VII)

wobei $R^1$, $R^2$, $R^3$, $R^4$, $R^5$, A, X, Y, m, n und z die oben genannte Bedeutung haben.

Die erfindungsgemäßen heterocyclisch substituierten Cycloalkano[b]-indolsulfonamide können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der heterocyclisch substituierten Cycloalkano[b]-indolsulfonamide können Metall- oder Ammoniumsalze der erfindungsgemäßen Stoffe, welche eine freie Carboxylgruppe besitzen, sein. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin oder Ethylendiamin.

Physiologisch unbedenkliche Salze können auch Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Heterocyclus steht im allgemeinen für einen 5- bis 7-gliedrigen, bevorzugt 5- bis 6-gliedrigen, gesättigten oder ungesättigten Ring, der als Heteroatome bis zu zwei Sauerstoff-, Schwefel- und/oder Stickstoffatome enthalten kann und an den weitere aromatische oder heterocyclische Ringe ankondensiert sein können.

Bevorzugt sind 5- und 6-gliedrige Ringe mit einem Sauerstoff, Schwefel und/oder bis zu zwei Stickstoffatomen, die auch benzokondensiert sein können.

Bevorzugt werden genannt: Thienyl, Furyl, Pyrrolyl, Pyrazolyl, Pyridyl, Pyrimidyl, Pyrazinyl, Pyridazinyl, Chinolyl, Isochinolyl, Chinazolyl, Chinoxazolyl, Cinnolyl, Thiazolyl, Benzothiazolyl, Isothiazolyl, Benzisothiazolyl, Oxazolyl, Benzoxazolyl, Isoxazolyl, Imidazolyl, Benzimidazolyl, Indolyl, Morpholinyl, Pyrrolidinyl, Piperidyl, Piperazinyl.

Die erfindungsgemäßen Stoffe zeigen überraschenderweise eine thrombozytenaggregationshemmende Wirkung, bewirken außerdem eine Hemmung der Thromboxansynthase an isolierten Plättchen und können zur therapeutischen Behandlung von Menschen und Tieren verwendet werden.

Bevorzugt sind Verbindungen der allgemeinen Formel (I)

in welcher

$R^1$, $R^2$, $R^3$, $R^4$ und $R^5$ gleich oder verschieden sind und

- für Wasserstoff, Fluor, Chlor, Brom, Nitro, Cyano, Trifluormethyl, Carboxy, Hydroxy oder Trifluormethoxy stehen,

- für eine Gruppe der Formel $-S(O)_wR^6$ stehen,
  worin
  $R^6$ - geradkettiges oder verzweigtes Alkyl mit bis zu 6 Kohlenstoffatomen oder Phenyl bedeutet, das seinerseits durch Fluor, Chlor, Brom, Nitro, Cyano oder Trifluormethyl substituiert sein kann,
  w - eine Zahl 0, 1 oder 2 bedeutet,
  oder
- für geradkettiges oder verzweigtes Alkoxy oder Alkoxycarbonyl mit jeweils bis zu 8 Kohlenstoffatomen stehen, oder
- für eine Gruppe der Formel $-NR^7R^8$ stehen,
  worin
  $R^7$ und $R^8$ gleich oder verschieden sind und
  - Wasserstoff, geradkettiges oder verzweigtes Alkyl mit bis zu 6 Kohlenstoffatomen oder Phenyl bedeuten,

  oder
- für Cyclopropyl, Cyclopentyl, Cyclohexyl oder Phenyl stehen, oder
- für geradkettiges oder verzweigtes Alkyl oder Alkenyl mit jeweils bis zu 8 Kohlenstoffatomen stehen, die gegebenenfalls durch Fluor, Chlor, Brom, Hydroxy, Carboxy, Cyano, Phenyl, geradkettiges oder verzweigtes Alkyl, Alkoxy oder Alkoxycarbonyl mit jeweils bis zu 6 Kohlenstoffatomen oder durch eine Gruppe der Formel $-S(O)_wR^6$ oder $-NR^7R^8$ substituiert sind,
  worin
  w, $R^6$, $R^7$ und $R^8$ die oben angegebene Bedeutung haben,
  oder
- für einen Rest oder eine Gruppe der Formel
  -D, -E-O-L-D, -E-NH-L-D, -E-CO-L-D oder -E-D stehen,
  worin
  D - Pyridyl, Chinolyl, Tetrazolyl, Benzthiazolyl, Isochinolyl, Benzimidazolyl, Pyrimidyl, Pyrrolyl, Thienyl, Furyl, Imidazolyl oder Thiazolyl bedeutet,
  und
  E und L gleich oder verschieden sind und
  - eine direkte Bindung oder
  - geradkettiges oder verzweigtes Alkyl oder Alkenyl mit jeweils bis zu 8 Kohlenstoffatomen bedeuten,

X

- für Phenyl steht, das gegebenenfalls bis zu 4-fach gleich oder verschieden durch Nitro, Hydroxy, Fluor, Chlor, Brom, Jod, Cyano, Trifluormethyl, Trifluormethoxy, Hydroxy, Carboxy, Phenyl, Phenoxy, geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert ist, oder
- für geradkettiges oder verzweigtes Alkyl mit bis zu 6 Kohlenstoffatomen oder für Trifluormethyl steht,

m - für die Zahl 1, 2 oder 3 steht,
n - für die Zahl 0 oder 1 steht,
z - für eine Zahl 1, 2 oder 3 steht,
A - für eine direkte Bindung oder für eine Gruppe der Formel

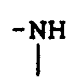

oder

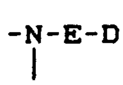

steht,
worin
D und E die oben angegebene Bedeutung haben,

Y
- für die Gruppe der Formel -CO-G steht,
  worin
  G - Hydroxy, Alkoxy mit bis zu 6 Kohlenstoffatomen, Phenoxy oder die Gruppe $-NHSO_2R^6$ bedeutet,
  worin
  $R^6$ die oben angegebene Bedeutung hat,
  oder
- für Tetrazolyl steht,

mit der Maßgabe, daß entweder der Substituent A für den Rest -N-E-D oder mindestens einer der Substituenten $R^1$, $R^2$, $R^3$, $R^4$ und $R^5$ für eine der Gruppen -D, -E-O-L-D, -E-NH-L-D, -E-CO-L-D oder -E-D stehen müssen,
gegebenenfalls in einer isomeren Form und ihre Salze.

Besonders bevorzugt sind Verbindungen der allgemeinen Formel (I),
in welcher

$R^1$, $R^2$, $R^3$, $R^4$ und $R^5$ gleich oder verschieden sind und
- für Wasserstoff, Fluor oder Chlor stehen,
- für eine Gruppe der Formel $-S(O)_w-R^6$ stehen,
  worin
  $R^6$ - geradkettiges oder verzweigtes Alkyl mit bis zu 4 Kohlenstoffatomen oder Phenyl bedeutet, das seinerseits durch Fluor, Chlor oder Brom substituiert sein kann
  und
  w - die Zahl 2 bedeutet,
  oder
- für geradkettiges oder verzweigtes Alkoxy oder Alkoxycarbonyl mit jeweils bis zu 4 Kohlenstoffatomen stehen, oder
- für die Gruppe der Formel $-NR^7R^8$ stehen,
  worin
  $R^7$ und $R^8$ gleich oder verschieden sind und
  - Wasserstoff, geradkettiges oder verzweigtes Alkyl mit bis zu 4 Kohlenstoffatomen oder Phenyl bedeuten,
  oder
- für geradkettiges oder verzweigtes Alkyl mit bis zu 6 Kohlenstoffatomen stehen, die gegebenenfalls durch Fluor, Chlor, Brom, Hydroxy, Cyano oder Phenyl substituiert sind,
  oder
- für einen Rest oder eine Gruppe der Formel
  -D, -E-O-L-D, -E-CO-L-D oder -E-D stehen,
  worin
  D - Pyridyl, Pyrimidyl, Imidazolyl oder Thiazolyl bedeutet,
  und
  E und L gleich oder verschieden sind und
  - eine direkte Bindung oder
  - geradkettiges oder verzweigtes Alkyl mit bis zu 6 Kohlenstoffatomen bedeuten,

X - für Phenyl steht, das gegebenenfalls bis zu 3-fach gleich oder verschieden durch Fluor, Chlor, Brom, Cyano, Trifluormethyl, Trifluormethoxy oder durch geradkettiges oder verzweigtes Alkyl, Alkoxy oder Alkoxycarbonyl mit jeweils bis zu 4 Kohlenstoffatomen substituiert ist,

m - für die Zahl 1 oder 2 steht,

n - für die Zahl 0 oder 1 steht,

z - für eine Zahl 1 oder 2 steht,

A - für eine direkte Bindung oder für eine Gruppe der Formel

-NH

oder

-N-E-D

steht,
worin
D und E die oben angegebene Bedeutung haben,

Y

- für die Gruppe der Formel -CO-G steht,
  worin
  G - Hydroxy, Alkoxy mit bis zu 4 Kohlenstoffatomen oder die Gruppe $-NHSO_2R^6$ bedeutet,
  worin
  $R^6$ die oben angegebene Bedeutung hat,
  oder
- für Tetrazolyl steht

mit der Maßgabe, daß entweder der Substituent A für den Rest

-N-E-D

oder mindestens einer der Substituenten $R^1$, $R^2$, $R^3$, $R^4$ und $R^5$ für eine der Gruppen -D, -E-O-L-D, -E-NH-L-D, -E-CO-L-D oder -E-D stehen müssen,
gegebenenfalls in einer isomeren Form und ihre Salze.

Die Verbindungen der allgemeinen Formel (I) können hergestellt werden, indem man
[A] im Fall, daß m für die Zahl 2 steht,
Verbindungen der allgemeinen Formel (VIII)

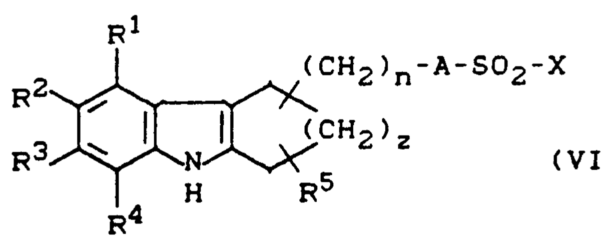

(VIII)

in welcher
$R^1$, $R^2$, $R^3$, $R^4$, $R^5$, z, n, A und X die oben angegebene Bedeutung haben,
mit Acrylnitril, gegebenenfalls in Anwesenheit einer Base in inerten Lösemitteln zu Verbindungen der allgemeinen Formel (IX)

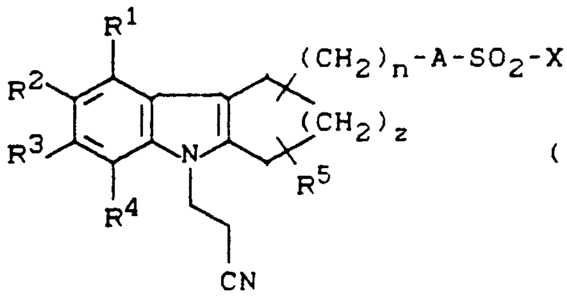

(IX)

in welcher
$R^1$, $R^2$, $R^3$, $R^4$, $R^5$, z, n, A und X die oben angegebene Bedeutung haben,
umsetzt,
im Fall, daß Y für den Tetrazolring steht, diese in Anwesenheit von Triethylaminhydrochlorid mit Natriumazid umsetzt,
oder im Fall der Herstellung der Säuren (Y = COOH) die Cyanogruppe nach üblicher Methode verseift,
im Fall der Herstellung der Ester (Y = $C_1$-$C_8$-Alkoxycarbonyl, Phenoxycarbonyl) die Säuren mit den entsprechenden Alkoholen in Gegenwart eines Katalysators gegebenenfalls in inerten Lösungsmitteln nach üblicher Methode umsetzt,
im Fall der Herstellung der Amide und Acylsulfonsäureamide (Y = $-CONR^7R^8$, $-CO-NHSO_2-R^6$) entweder die Ester oder die Säuren nach üblicher Aktivierung, mit den Aminen oder Sulfonsäureamiden der allgemeinen Formeln (Xa) und (Xb)

$HNR^7R^8$ (Xa)

$NH_2SO_2-R^6$ (Xb)

in welcher
$R^6$, $R^7$ und $R^8$ die oben angegebene Bedeutung haben,
gegebenenfalls in Anwesenheit eines Katalysators umsetzt,
oder indem man
[B] Verbindungen der allgemeinen Formel (XI)

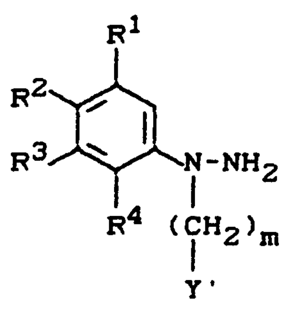

(XI)

in welcher
$R^1$, $R^2$, $R^3$, $R^4$, und m die oben angegebene Bedeutung haben,
und
Y' - für ($C_1$-$C_4$)-Alkoxycarbonyl oder Cyano steht,
mit Cycloalkanonsulfonamiden der allgemeinen Formel (XII)

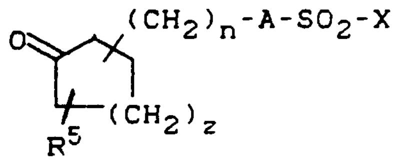

(XII)

in welcher
z, n, A, $R^5$ und X die oben angegebene Bedeutung haben,
in inerten Lösemitteln, gegebenenfalls in Anwesenheit eines Katalysators umsetzt,
im Fall der Herstellung der Säuren (Y = COOH) die Ester oder Cyanogruppe nach üblicher Methode verseift,
im Fall der Herstellung von Estern (Y = $C_1$-$C_8$-Alkoxycarbonyl, Phenoxycarbonyl) die Säuren mit den entsprechenden Alkoholen in Gegenwart eines Katalysators gegebenenfalls in inerten Lösemitteln nach üblicher Methode umsetzt, oder Ester nach üblichen Methoden umestert,
im Fall der Herstellung der Amide und Sulfonsäureamide (Y = -CONR$^7$R$^8$, -CO-NHSO$_2$-R$^6$) entweder direkt die Ester oder die Säuren nach üblicher Aktivierung, mit den Aminen oder Sulfonsäureamiden der allgemeinen Formeln (Xa) und (Xb)
gegebenenfalls in Anwesenheit eines Katalysators umsetzt,
im Fall, daß Y für den Tetrazolring steht, die Cyanoverbindung (Y = CN) in Anwesenheit von Triethylaminhydrochlorid mit Natriumazid umsetzt,
oder indem man
[C] in Verbindungen der allgemeinen Formel (Ia)

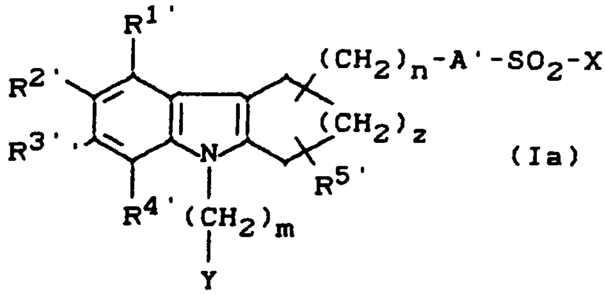

(Ia)

in welcher
$R^{1'}$, $R^{2'}$, $R^{3'}$, $R^{4'}$ und $R^{5'}$ die angegebene Bedeutung haben, jedoch nicht für eine Gruppe der Formel -D, -E-O-L-D, -E-NH-L-D, -E-CO-L-D oder -ED steht,
A' für die Gruppe -NH- steht und
m, Y, X und z die angegebene Bedeutung haben,
die Reste der Formel -D, -E-O-L-D, -E-NH-L-D, -E-CO-L-D oder E-D nach üblichen Methoden, beispielsweise durch nucleophile oder elektrophile aromatische Substitution einführt, und gegebenenfalls Carbonylgruppen nach üblichen Methoden zu Methylengruppen reduziert,
im Falle der Herstellung der Säuren (Y = -COOH) die Ester nach üblicher Methode verseift,
im Fall der Variation der Ester (Y = $C_1$-$C_8$-Alkoxycarbonyl, Phenoxycarbonyl) die Säuren mit den entsprechenden Alkoholen in Gegenwart eines Katalysators gegebenenfalls in inerten Lösemitteln nach üblicher Methode umsetzt,
im Fall der Amide und Acylsulfonamide (Y = -CONR$^7$R$^8$, -CO-NHSO$_2$-R$^6$) entweder direkt die Ester oder die Säuren nach üblicher Aktivierung, mit den Aminen oder Sulfonsäureamiden der allgemeinen Formeln (Xa) und (Xb) gegebenenfalls in Gegenwart eines Katalysators umsetzt,
und dann im Fall der heterocyclisch substituierten Cycloalkano[b]-dihydroindolsulfonamide die nach Verfahren [A], [B] oder [C] hergestellten heterocyclisch substituierten Indolsulfonamide in Gegenwart eines Reduktionsmittels in inerten Lösemitteln reduziert,
gegebenenfalls die Isomeren trennt und
im Fall der Herstellung der Salze mit einer entsprechenden Base oder Säure umsetzt.

Das erfindungsgemäße Verfahren kann durch folgendes Formelschema beispielhaft erläutert werden:

[A]

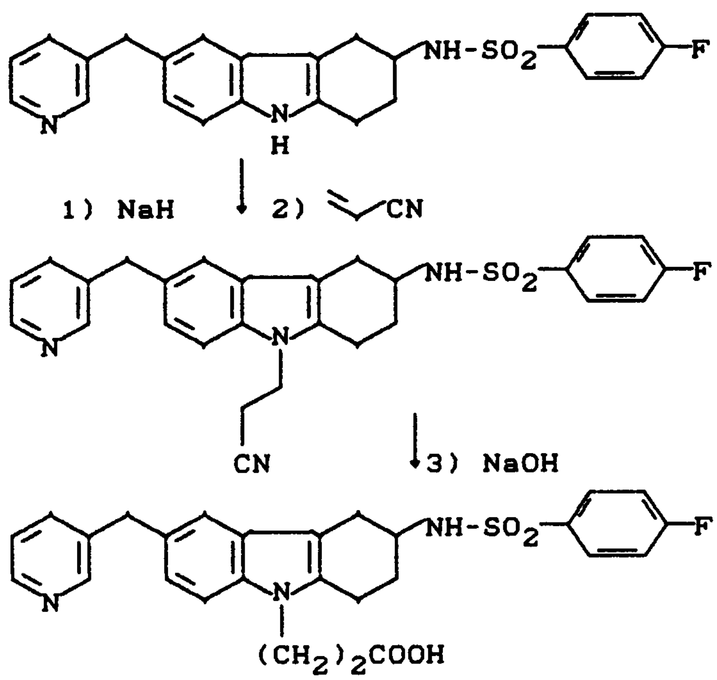


[B]

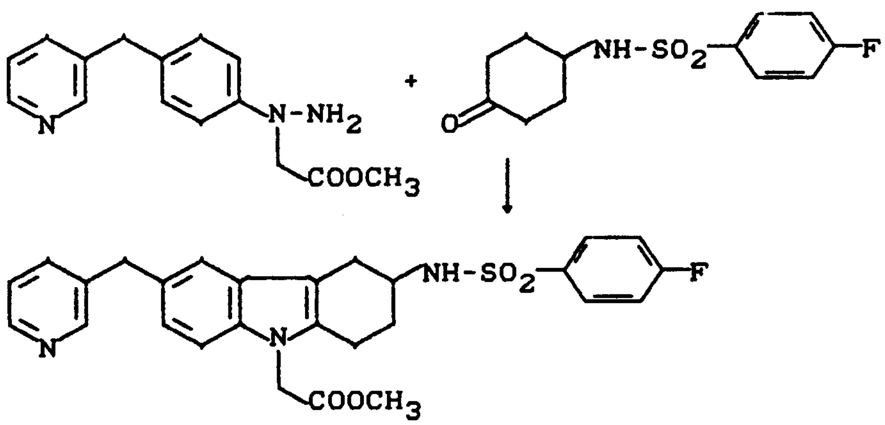

[C]

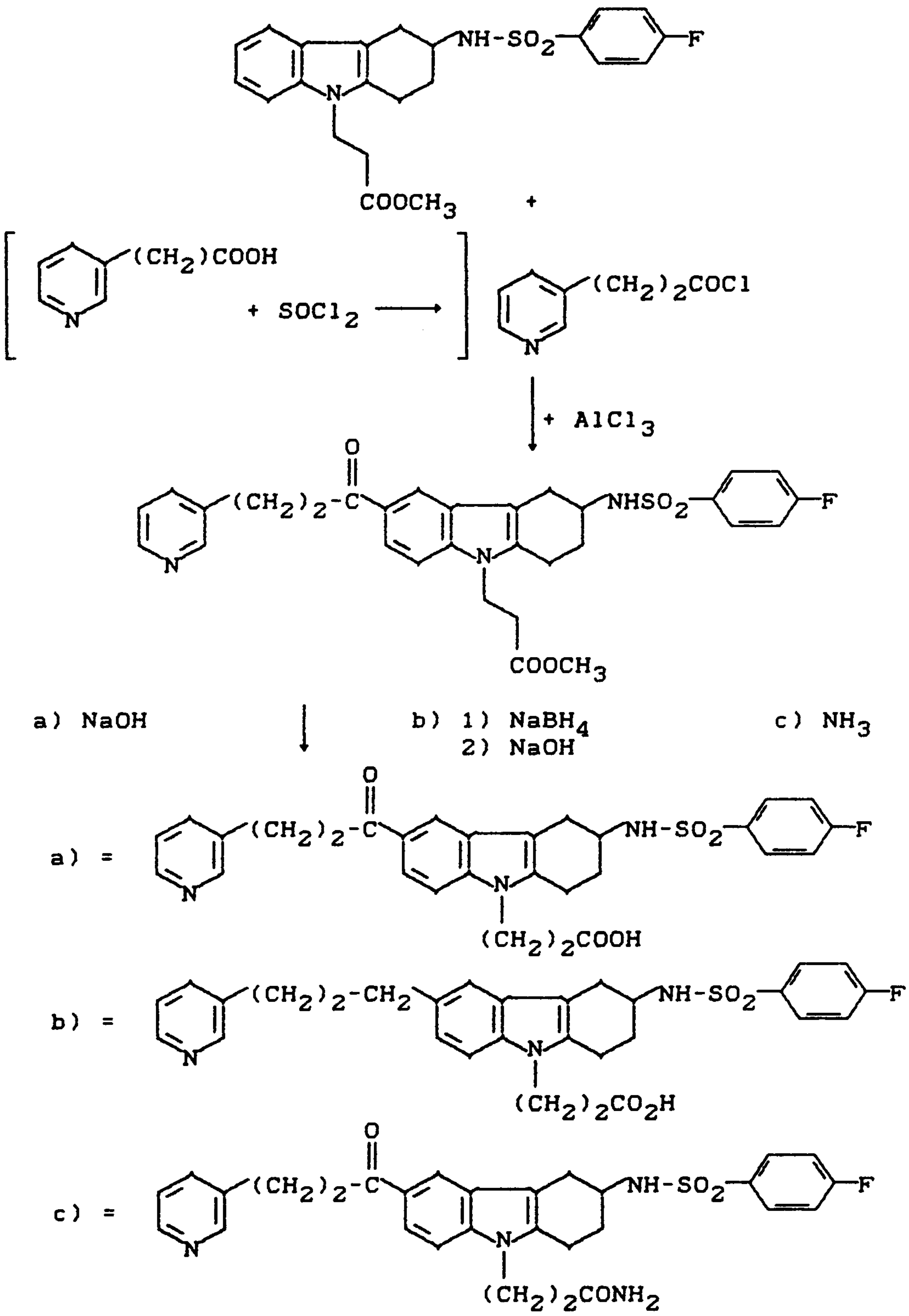


Lösungsmittel für die erfindungsgemäßen Verfahren [A] und [B] können Wasser und organische Lösungsmittel sein, die sich unter den Reaktionsbedingungen nicht verändern. Hierzu gehören bevorzugt chlorierte Kohlenwasserstoffe, wie beispielsweise Chloroform oder Methylenchlorid, Alkohole wie Methanol, Ethanol, Propanol oder Isopropanol, Ether wie Diethylether, Tetrahydrofuran, Dioxan, Glykolmono-oder -dimethylether, Kohlenwasserstoffe wie Benzol, Toluol, Xylol, Cyclohexan, Hexan oder Erdölfraktionen, Dimethylsulfoxid, Dimethylformamid, Hexamethylphosphorsäuretriamid, Essigester, Acetonitril oder Pyridin.

Ebenso ist es möglich, Gemische der genannten Lösungsmittel zu verwenden.

Basen für die erfindungsgemäßen Verfahren [A] und [B] können übliche basische Verbindungen sein. Hierzu gehören vorzugsweise Alkali- und Erdalkalihydroxide wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat-, -ethanolat oder Kaliumtert.-butylat oder Amide wie Natriumamid oder Lithiumdiisopropylamid, oder organische Amine oder Ammoniumsalze wie Benzyltrimethylammoniumhydroxid, Tetrabutylammoniumhydroxid, Pyridin, Triethylamin oder N-Methylpiperidin.

Die erfindungsgemäßen Verfahren [A] und [B] werden im allgemeinen in einem Temperaturbereich von 0°C bis 150°C, bevorzugt von 0°C bis 100°C, durchgeführt.

Im allgemeinen werden die Verfahren [A] und [B] bei Normaldruck durchgeführt. Es ist aber auch möglich, bei Unterdruck oder bei Überdruck zu arbeiten (z.B. von 0,5 bis 5 bar).

Die Verseifung der Ester erfolgt nach üblicher Methode, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +140°C, bevorzugt von +20°C bis +100°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 5 mol, bevorzugt von 1 bis 2 mol bezogen auf 1 mol des Esters eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren- Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Lösungen der Carboxylate mit den oben aufgeführten Säuren auch die Salze dieser Heterocyclen mit den anorganischen Säuren gewonnen werden.

Die Veresterung der Säuren erfolgt nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dieser Alkohol auch als Lösemittel eingesetzt.

Als Katalysatoren können anorganische Säuren, wie beispielsweise Schwefelsäure oder anorganische Säurechloride, wie beispielsweise Thionylchlorid, eingesetzt werden.

Im allgemeinen setzt man 0,01 bis 1, bevorzugt 0,05 bis 0,5 mol Katalysator bezogen auf 1 mol Reaktionspartner ein.

Die Amidierung erfolgt in einem der oben angegebenen Lösemittel, bevorzugt in Alkoholen wie Ethanol oder Methanol, in einem Temperaturbereich von 0°C bis +50°C, bevorzugt von +10 bis +30°C, und bei Normaldruck.

Sowohl die Veresterung als auch die Amidierung können gegebenenfalls über die aktivierte Stufe der Säurehalogenide (I, Y = CO-Halogen), die aus der entsprechenden Säure durch Umsetzung mit Thionylchlorid, Phosphortrichlorid, Phosphorpentachlorid, Phosphortribromid oder Oxalylchlorid hergestellt werden können, verlaufen.

Als Lewissäuren eignen sich $BF_3$, Aluminiumhalogenide, wie $AlCl_3$, $ZnCl_2$ oder $Ag^{\oplus}$-Salze. Bevorzugt sind $BF_3$ und $AlCl_3$.

Die Verbindungen der allgemeinen Formel (VIII) sind neu und können hergestellt werden,
indem man substituierte Aniline der allgemeinen Formel (XIII)

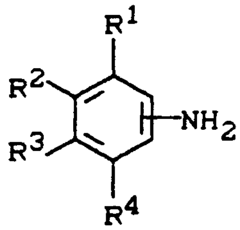

(XIII)

in welcher
$R^1$, $R^2$, $R^3$ und $R^4$ die oben angegebene Bedeutung haben,
in einer Zweistufenreaktion zunächst mit Natriumnitrit in Anwesenheit von Säuren und nachfolgend Natriumbisulfit in die entsprechenden Hydrazine der allgemeinen Formel (XIV)

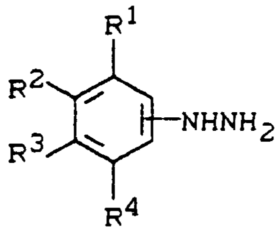

(XIV)

in welcher
$R^1$, $R^2$, $R^3$ und $R^4$ die oben angegebene Bedeutung haben,
überführt und anschließend
mit Cycloalkanonsulfonamiden der allgemeinen Formel (XII)

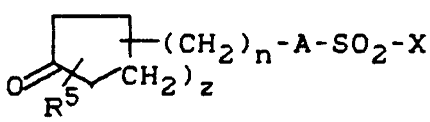

(XII)

in welcher
$R^5$, z, n, A und Z die oben angegebene Bedeutung haben,
in Gegenwart von den oben aufgeführten inerten Lösemitteln, gegebenenfalls in Anwesenheit eines Katalysators, umsetzt.

Die Verbindungen der allgemeinen Formel (IX) sind ebenfalls neu und können wie oben angegeben hergestellt werden.

Die Umsetzung mit Phenylhydrazinen der Formel (XIV) verläuft unter den bei Verfahren [B] beschriebenen Reaktionsbedingungen.

Als Säuren bei der Hydrazindarstellung (1. Schritt) eignen sich anorgansiche Säuren oder deren Salze, wie beispielsweise Salzsäure oder Schwefelsäure.

Die substituierten Aniline der allgemeinen Formel (XIII) sind an sich bekannt oder können über die Stufe der jeweiligen Nitroverbindungen nach literaturbekannten Reduktionsmethoden, beispielsweise mit Wasserstoff an Pd/C hergestellt werden [vgl. EP 69 521; J. Med. Chem. 19, 1079 (1976); J. Heterocycl. Chem. 21, 1849, (1984)].

Die Hydrazine der allgemeinen Formel (XIV) sind teilweise bekannt und können aber nach der oben aufgeführten Methode hergestellt werden.

Die Cycloalkanonsulfonamide der allgemeinen Formel (XII) sind teilweise bekannt ($R^5$ = H) oder neu und können in diesem Fall in Analogie zu dem in der DOS 36 31 824 publizierten Verfahren und zusätzlicher Einführung des Substituenten ($R^5 \neq$ H) nach üblicher Methode hergestellt werden.

Die erfindungsgemäßen enantiomerenreinen Verbindungen der allgemeinen Formel (I) können nach üblichen Methoden, z.B. in Analogie zu dem in der DOS 36 31 824 beschriebenen Verfahren erhalten werden.

Die Verbindungen der allgemeinen Formel (XI) sind teilweise bekannt oder können in Analogie zu

literaturbekannten Methoden hergestellt werden [vgl. z.B. DOS 23 12 256].

Die Amine der allgemeinen Formel (Xa) sind teilweise bekannt [vgl. Houben-Weyl, "Methoden der organischen Chemie", Bd. XI/1 und XI/2].

Die Sulfonsäureamide der allgemeinen Formel (Xb) sind teilweise bekannt [vgl. Beilstein, 11, 26].

Die heterocyclisch substituierten Cycloalkano[b]-indolsulfonamide bzw. deren Salze sowie Isomere können als Wirkstoffe in Arzneimitteln eingesetzt werden. Die Stoffe weisen eine thrombozytenaggregationshemmende und Thromboxan $A_2$-antagonistische Wirkung auf und hemmen die Thromboxansynthase an isolierten Plättchen. Sie können zur Behandlung von thromboembolischen Erkrankungen und Ischämien wie Myokardinfarkt, Hirnschlag, transitorisch und ischämische Attacken, Angina pectoris, periphere Durchblutungsstörungen, Verhinderung von Restenosen wie nach Thrombolysetherapie, percutanen transluminalen Angioplastien (PTA), percutanen transluminalen Koronarangioplastien (PTCA), Bypass sowie zur Behandlung von Arteriosklerose, Asthma und Allergien eingesetzt werden.

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreiches Zitratplasma (PRP)[1] (Jürgens/ Beller, Klinische Methoden der Blutgerinnungsanalyse; Thieme Verlag, Stuttgart, 1959).

Für diese Untersuchungen wurden 0,8 ml PRP und 0,1 ml der Wirkstofflosung bei 37°C im Wasserbad vorinkubiert. Anschließend wurde die Thrombozytenaggregation nach der turbidometrischen Methode (Born, G.V.R., J. Physiol. (London), <u>162</u>, 67, 1962) im Aggregometer bei 37°C bestimmt (Therapeutische Berichte <u>47</u>, 80-86, 1975). Hierzu wurde die vorinkubierte Probe mit 0,1 ml Kollagen, einem aggregationsauslösenden Agens, versetzt. Die Veränderung der optischen Dichte in der Probe der PRP1 wurde während einer Zeitdauer von 6 Minuten aufgezeichnet und der Ausschlag nach 6 Minuten bestimmt. Hierzu wird die prozentuale Hemmung gegenüber der Kontrolle errechnet. Als Grenzkonzentration wird der Bereich der minimal effektiven Konzentration angegeben.

| Beispiel-Nr. | TAH-Grenzkonzentration (µg/ml) |
|---|---|
| 17 | 0,03 - 0,1 |
| 18 | 0,3 - 1 |
| 20 | 0,03 - 0,1 |
| 25 | 0,03 - 0,1 |

Messung der Thromboxansynthase an gewaschenen Humanthrombozyten

1. <u>Präparation von Thrombozytensuspensionen</u>

Blut von gesunden Spendern wird in EDTA (1% in 0,9 % NaCl, 9 + 1) aufgenommen und bei 1000 U/min (150 g) 20 min zentrifugiert. Das plättchenreiche Plasma (PRP)[2] wird abgehebert und jeweils 10 ml bei 2500 U/min 20 min zentrifugiert. Das plättchenreiche Plasma[2] wird abdekantiert. Die verbleibenden Plättchen werden in 5 ml Resuspensionspuffer (0,15 M TRIS / 0,9% NaCl/ 77 mmol EDTA, 8:91:1; mit 1 N HCl auf pH 7,4 eingestellt) suspendiert, 20 min bei 2500 U/min zentrifugiert und in 1 ml Resuspensionspuffer suspendiert. Die Thrombozytenzahl wird auf 3 x $10^5$/µl eingestellt.

2. <u>Messung der Thromboxansynthase</u>

1 ml der Plättchensuspension und 0,01 ml des Prüfpräparates in 10% DMSO, werden 2 min bei 37°C inkubiert. Dazu werden 0,1 ml $^3$H-Arachidonsäure Amersham Buchler GmbH und Co. KG (6,6 x $10^{-5}$ mol/1) mit einer spezifischen Aktivität von 140 MBq/mmol zugegeben und weitere 10 min bei 37°C inkubiert. Nach der Reaktion wird das Gemisch mit ca. 0,02 ml 0,5 N Citronensäure angesäuert und unmittelbar mit 3-mal mit je 1 ml Essigester extrahiert. Die Überstände werden in 10 ml Glasröhrchen gesammelt und der Essigester unter $N_2$ bei 25°C abgeblasen. Der Rückstand wird in 50 µl MeOH/$CHCl_3$ (1:1) aufgenommen und auf DC-Glasplatten (Kieselgel 60 F254 20 x 20 cm, Merck) aufgetragen.

Die Trennung erfolgt in einem Fließmittelgemisch $CHCl_3$/MeOH/Eisessig/$H_2O$ (80:8:1:0,8). Die Verteilung der Radioaktivität wird in einem DC-Scanner Ramona-Ls der Fa. Raytest erfaßt und mit einem Integrationsprogramm quantitativ ausgewertet.

Bestimmt wird die Konzentration der Prüfsubstanzen, die zu einer 50% Hemmung der Thromboxanbildung gegenüber der Kontrolle führt.

| Beispiel Nr. | $IC_{50}$ mol/l |
|---|---|
| 11 | $2 \times 10^{-7}$ |
| 17 | $1 \times 10^{-6}$ |
| 24 | $1 \times 10^{-7}$ |

Thromboxanrezeptorbindungstest an menschlichen Thrombozytenmembranen

a) Membranpräparation

Das am Vorabend nach Standardmethoden abgenommene Blut wurde morgens 10 min bei 10°C mit 2800 U/min zentrifugiert. Der dabei als Schicht zwischen dem plättchenarmen Plasma und den Erythrozyten entstandenen Buffy-Coat wurde mit 10 µM Indomethazin versetzt. Aus dem Buffy-Coat wurden nach einer Methode, die von Barber und Jamieson (vgl. Barber, A.J., Jamieson, G.A: Isolation and characterization of plasma membranes from humanblood platelets, J Biol. Chem. 245, 6357-6365, 1970), beschrieben wurde, Trombozytenmembranen präpariert. Als wichtigster Schritt werden dabei Trombozyten mit Glycerin beladen und durch osmotischen Schock zur Lyse gebracht.

Die so erhaltenen, gewaschenen Membranen wurden in Tris-NaCl-Glucose-Puffer (50 mM Tris, 100 mM NaCl, 5 mM Glucose, pH 7,4) resuspendiert, in Trockeneis schockgefroren und bei -70°C gelagert.

b) Verdrängungsstudien

Für die Verdrängungsstudien wurden 100 µg Membranprotein und ca. 5 nM $^3$H-(3R)-3-(4-Fluorphenylsulfonamido)-9-(2-carboxyethyl)-1,2,3,4-tetrahydro-carbazol [zur Herstellung vgl. DOS 36 31 824; die radioaktive Markierung erfolgt nach literaturbekannter Methode] in einem Gesamtvolumen von 1 ml Tris-NaCl-Glucose-Puffer inkubiert. Zum Ansatz wurden steigende Konzentrationen der verdrängenden unmarkierten erfindungsgemäßen Verbindungen gelöst in DMSO zugegeben (Endkonzentration, 0,5% DMSO, bezogen auf das Assay Volumen).

Die Substanzkonzentration $IC_{50}$, die benötigt wird, um 50% der spezifischen Bindung zu verdrängen, wurde mit Hilfe eines logit-log Plots nach HILL bestimmt. Aus der $IC_{50}$ sowie der Dissoziationskonstanten $K_D$ (bestimmt durch Scatchard-Analyse) wurde die Inhibitionskonstante $K_I$ bestimmt.

Zur vorliegenden Erfindung gehören auch pharmazeutische Zubereitungen, die neben inerten, nichttoxischen, pharmazeutisch geeigneten Hilfs- und Trägerstoffen eine oder mehrere Verbindungen der allgemeinen Formel (I) enthalten, oder die aus einem oder mehreren Wirsktoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs-oder Trägerstoffen.

Im allgemeinen hat es sich als vorteilhaft erwiesen, den oder die Wirkstoffe der Formel (I) in Gesamtmengen von etwa 0,03 bis etwa 30 mg/kg, bevorzugt bis etwa 5 mg/kg Körpergewicht je 24 Stunden, gegebenenfalls in Form mehrerer Einzelgaben, zur Erzielung des gewünschten Ergebnisses zu verabreichen.

Eine Einzelgabe enthält den oder die Wirkstoffe vorzugsweise in Mengen von 0,01 bis etwa 10, besonders bevorzugt 0,1 bis 1,0 mg/kg Körpergewicht.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Ausgangsverbindungen

Beispiel I

3-Pyridylpropionsäurechlorid-Hydrochlorid

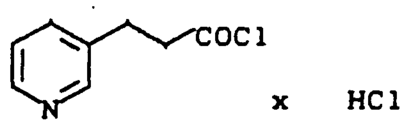

10,1 g (66,8 mmol) 3-Pyridylpropionsäure [vgl. F.A. Walker und M. Benson, J. Am. Chem. Soc. 102, 5530 (1980)] werden in 30 ml Dichlormethan p.a. aufgeschlämmt und langsam mit 10,83 g (91 mmol) Thionylchlorid versetzt. Man läßt 24 h unter Rückfluß kochen, kühlt ab und saugt den entstandenen Niederschlag ab. Der Feststoff wird mit kaltem Dichlormethan und Ether gewaschen und im Hochvakuum vom Restlösemittel befreit.
Ausbeute: 12,13 g (58,9 mmol)
Schmp.: 159- 161°C

Beispiel II

3-(Hydroxy-(3-nitrophenyl)-methyl)-pyridin

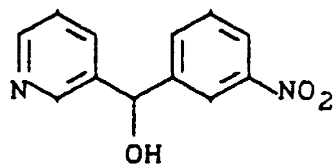

Literaturanalog [R.N. Carde, P.C. Hayes, G. Jones und C.J. Cliff, J. Chem. Soc., Perkin Trans. I, 1981, 1132] setzt man 30,0 g (189,8 mmol) 3-Brompyridin in 90 ml absolutem Ether bei -70°C langsam mit 64 ml einer 2,5 molaren Lösung von n-Butyllithium in Ether um, rührt nach beendeter Zugabe 15 Minuten nach und tropft dann eine Lösung von 29,0 g (192,0 mmol) 3-Nitrobenzaldehyd in 200 ml Tetrahydrofuran abs. zu. Die Reaktionsmischung wird 2 Stunden bei -70°C und 2 Stunden bei Raumtemperatur nachgerührt und dann bei 0°C mit Wasser und 40%igem Natriumhydrogensulfat verrührt. Die wäßrige Phase wird mehrfach mit Ether extrahiert, die organischen Phasen dann mit Natriumsulfat getrocknet und einrotiert. Eine Säulenchromatographie (Kieselgel 60, Merck, 40-63 μm, Laufmittel: zuerst Toluol:Essigester = 1:1 später 1:4) liefert 21,3 g (92,5 mmol) Produkt.
DC (Toluol:Essigester = 1:4)
$R_f$ = 0,22

Beispiel III

3-(3-Aminobenzyl)-pyridin

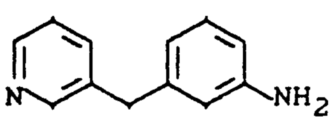

14,85 g (64,5 mmol) der Verbindung aus Beispiel II werden an 7,4 g Palladium (10%ig auf Tierkohle) in 500 ml Ethanol und 18 ml konzentrierter Salzsäure bei Raumtemperatur unter Normaldruck $H_2$ hydriert. Nach 18 Stunden wird der Katalysator abfiltriert, die Lösung mit wäßriger 25%iger Ammoniaklösung auf pH 10 gestellt und die erhaltene Mischung zur Trockne einrotiert. Der Feststoff wird mit Ethanol : Ether = 4:1 ausgerührt, abfiltriert und das Filtrat eingedampft.
Ausbeute: 10,21 g (55,4 mmol)
DC (Dichlormethan : Methanol = 10:1)

$R_f$ = 0,61

Beispiel IV

3-(4-Hydrazinobenzyl)-pyridin

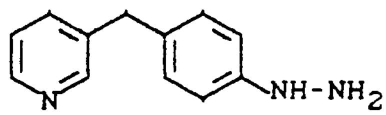

6,8 g (36,9 mmol) 3-(4-Aminobenzyl)-pyridin [EP 69 521] werden in 9,2 ml konzentrierter Salzsäure und 12 ml Wasser gelöst, auf 5°C gekühlt und mit einer Lösung von 2,55 g (36,9 mmol) Natriumnitrit in 17 ml Wasser umgesetzt. Man rührt 30 Minuten bei 0-5°C nach, und tropft die Reaktionsmischung dann bei Raumtemperatur zu 120 g einer 40%igen Natriumhydrogensulfitlösung, die mit Natriumhydroxid auf einen pH-Wert von 6,5 gestellt wurde, Anschließend kocht man die Mischung 4 Stunden unter Rückfluß, wobei der pH-Wert von 6,5 stets eingehalten wird. Man kühlt ab und gießt den Ansatz in 120 ml Natronlauge, der pH-Wert liegt dann bei 12. Nach Extraktion mit Dichlormethan und Trocknung mit Natriumsulfat rotiert man ein und erhält 4,66 g (23,4 mmol) Produkt.
DC: (Toluol:Aceton = 2:1)
$R_f$ = 0,50

Analog Beispiel IV wurden die in Tabelle 1 aufgeführten Verbindungen erhalten:

Tabelle 1

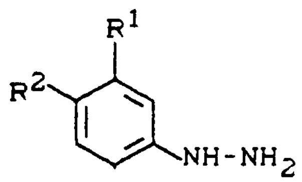

| Bsp.-Nr. | $R^1$ | $R^2$ | $R_f$ |
|---|---|---|---|
| V | H | $-CH_2-$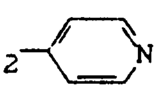 | 0,39[a)] |
| VI | $-CH_2-$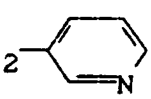 | H | 0,41[a)] |
| VII | H | $-CH_2-$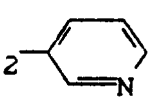 | 0,50[a)] |
| VIII | H | $-(CH_2)_2-$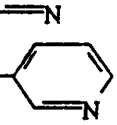 | 0,46[b)] |
| IX | 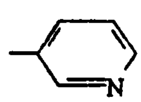 | H | 0,22[c)] |

a) (Toluol:Aceton = 2:1)
b) (Dichlormethan:Methanol = 10:1)
c) (Essigester:Toluol = 4:1)

Beispiel X

9-(2-Cyano-ethyl)-6-hydroxy-3-(4-fluorphenylsulfonamido)-1,2,3,4-tetrahydrocarbazol

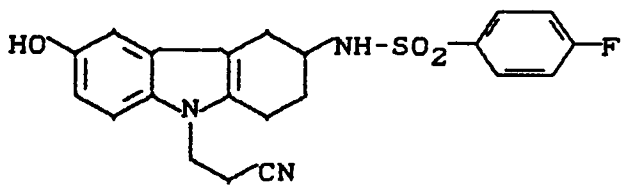

17,18 g (40,2 mmol) 9-(2-Cyano-ethyl)-3-(4-fluorphenylsulfonamido)-6-methoxy-1,2,3,4-tetrahydrocarbazol werden unter Stickstoff in 200 ml Dichlormethan p.a. gelöst und bei -78°C mit 92,5 ml einer 1 M Lösung von Bortribromid in Dichlormethan umgesetzt. Man rührt 30 Minuten bei - 78°C, läßt über 30 Minuten auf Raumtemperatur kommen und rührt 1 Stunde nach. Darauf hydrolysiert man mit 1,5 l Wasser und stellt mit 2 N Schwefelsäure einen pH-Wert von 1 ein. Mehrmalige Extraktion der wäßrigen Phase mit Essigester und Trocknung der organischen Phase mit Natriumsulfat liefert nach Abrotieren des Lösemittels ein Rohprodukt, das säulenchromatographisch aufgereinigt wird (Kieselgel 60, Merck, 40-63 µm, Toluol:Essigester = 3:2).
DC (Toluol:Essigester = 3:2)
$R_f$ = 0,39

Beispiel XI

(3R)-(4-Fluorphenylsulfonamido)-9-(2-methoxycarbonylethyl)-1,2,3,4-tetrahydrocarbazol

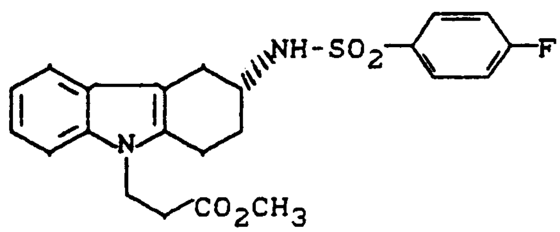

10,0 g (24 mmol) (3R)-(4-Fluorphenylsulfonamido)-9-(2-carboxy-ethyl)-1,2,3,4-tetrahydrocarbazol [vgl. DOS 36 31 824] werden in 100 ml absolutem Methanol mit 5 ml konzentrierter Schwefelsäure 2 Stunden unter Rückfluß gekocht. Nach dem Abkühlen neutralisiert man mit gesättigter Natriumhydrogencarbonatlösung, rotiert den Alkoholanteil ab, füllt mit Wasser bis zum alten Volumen auf und extrahiert mehrfach mit Dichlormethan. Die organische Phase wird mit Natriumsulfat getrocknet, eingedampft und im Hochvakuum vom Restlösemittel befreit.
Ausbeute: 10,07 g (22,7 mmol)
DC: $R_f$ = 0,52 (Toluol:Ethanol = 6:1)

Entsprechend läßt sich das analoge racemische 6-Hydroxyderivat aus der entsprechenden Carbonsäure gewinnen:
DC: $R_f$ = 0,59 (Toluol:Essigester = 1:1)

Beispiel XII

3-(4-Fluorphenylsulfonamido)-6-methoxy-1,2,3,4-tetrahydrocarbazol

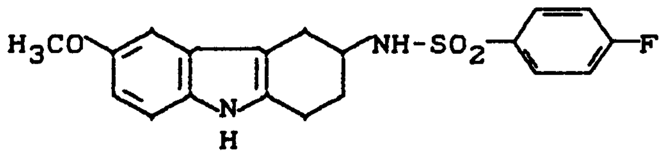

Die Titelverbindung wird in Analogie zur später aufgeführten Vorschrift des Beispiels XVI hergestellt. $R_f$ = 0,32 (Toluol : Essigester 4:1)

Beispiel XIII

9-(2-Cyanoethyl)-3-(4-fluorphenylsulfonamido)-6-methoxy-1,2,3,4-tetrahydrocarbazol

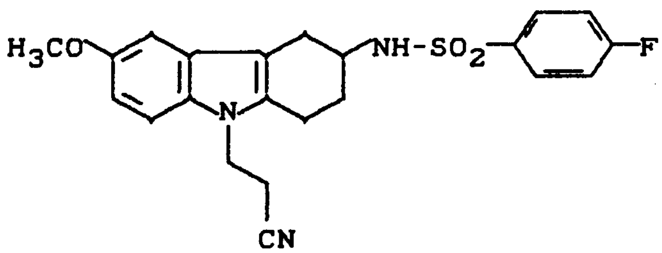

Die Titelverbindung wird in Analogie zur später aufgeführten Vorschrift des Beispiels XXIV hergestellt. $R_f$ = 0,22 (Toluol : Essigester = 4:1)

Beispiel XIV

3-(4-Fluorphenylsulfonamido)-6-hydroxy-9-(2-carboxyethyl)-1,2,3,4-tetrahydrocarbazol

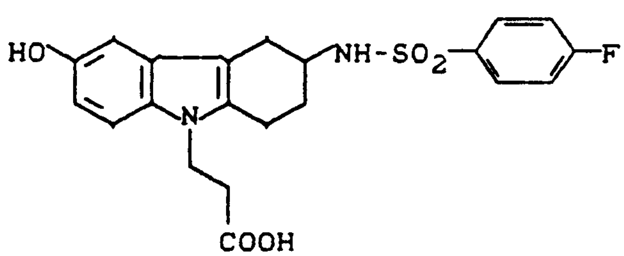

Die Titelverbindung wird in Analogie zur später aufgeführten Vorschrift des Beispiels 11 hergestellt. $R_f$ = 0,59 (Toluol:Essigester 1:4)

Beispiel XV

3-(4-Chlorphenylsulfonamido)-6-fluor-9-(2-(5-tetrazolyl)ethyl)-1,2,3,4-tetrahydrocarbazol

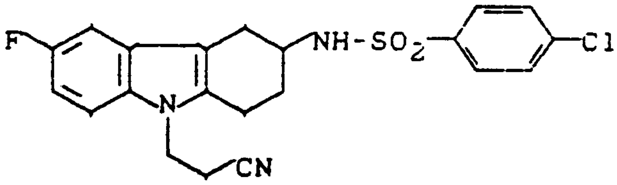

Die Titelverbindung wird in Analogie zur später beschriebenen Vorschrift des Beispiels XXIV hergestellt.
$R_f$ = 0,60 (Dichlormethan : Essigester = 10:1)

Beispiel XVI

3-(4-Fluorphenylsulfonamido)-6-(3-pyridylmethyl)-1,2,3,4-tetrahydrocarbazol-Hydrochlorid

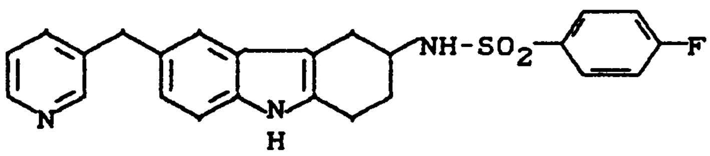

4,66 g (23,4 mmol) der Verbindung aus Beispiel IV werden mit 6,25 g (23,4 mmol) 4-(4-Fluorphenylsulfonamido)-cyclohexanon [vgl. DE 36 31 824] in 96 ml Ethanol und 5 ml konzentrierter Schwefelsäure 8 Stunden unter Rückfluß gekocht. Darauf kühlt man ab, neutralisiert mit Natriumbicarbonatlösung und extrahiert mehrfach mit Essigester. Die organische Phase wird mit Natriumsulfat getrocknet, einrotiert und im Hochvakuum vom Restlösemittel befreit. Nach Säulenchromatographie (Kieselgel 60, Merck 40-63 µm, Essigester:Toluol = zuerst 1:2, dann 1:1, zuletzt 2:1) fallen 7,5 g verunreinigtes Produkt an, das in Dichlormethan gelöst und mit Chlorwasserstoffgas in sein Hydrochlorid überführt wird. Man rotiert ein und rührt mit Ether/Petrolether (30-50°C) aus. Nach Absaugen und Trocknen im Hochvakuum erhält man 6,87 g (14,6 mmol) Produkt.
DC (Essigester:Toluol = 4:1)
$R_f$ = 0,55

In Analogie zu Beispiel XVI lassen sich die in Tabelle 2 aufgeführten Verbindungen herstellen:

Tabelle 2

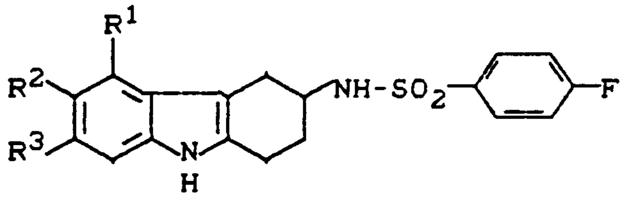

| Bsp.-Nr. | $R^1$ | $R^2$ | $R^3$ | $R_f$ |
|---|---|---|---|---|
| XVII | H | $-CH_2$ 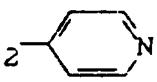 | H | 0,25[a)] |
| XVIII | H | $-CH_2$ 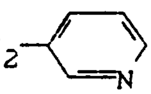 | H | 0,55[b)] |
| XIX | H | H | $-CH_2$ 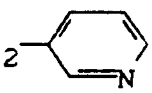 | 0,33[c)] |
| XX | $-CH_2$ 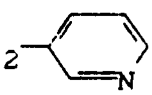 | H | H | 0,49[c)] |
| XXI | H | $-(CH_2)_2$ 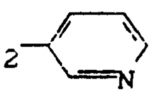 | H | 0,42[c)] |
| XXII | 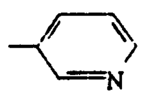 | H | H | 0,41[c)] |
| XXIII | H | H | 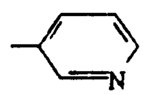 | 0,24[c)] |

Laufmittelgemische:

a) Essigester:Toluol = 1:1

b) Essigester:Toluol = 4:1

c) Essigester:Toluol = 2:1

Beispiel XXIV

9-(2-Cyanoethyl)-3-(4-fluorphenylsulfonamido)-6-(3-pyridylmethyl)-1,2,3,4-tetrahydro-carbazol

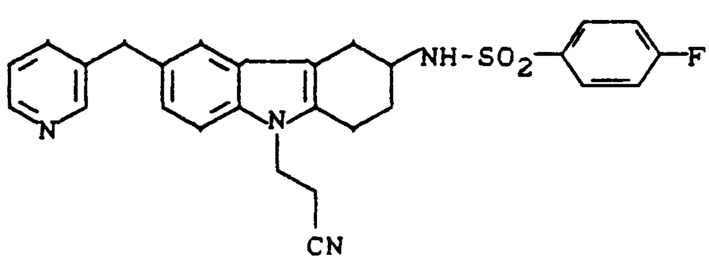

4,95 g (10,5 mmol) der Verbindung aus Beispiel XVI werden in 100 ml Dimethylformamid p.a. gelöst, mit 0,693 g (23,1 mmol) Natriumhydrid (mit 20% Paraffinöl stabilisiert) deprotoniert und dann bei Raumtemperatur mit 1,5 ml (22,8 mmol) Acrylnitril umgesetzt. Nach 2 h ist die Umsetzung unvollständig, daher setzt man 0,071 g (2,4 mmol) Natriumhydrid und 0,7 ml (10,6 mmol) Acrylnitril zu; nach 1 Stunde ist die Reaktion vollständig abgelaufen. Man versetzt mit Wasser und extrahiert mit Essigester; unlösliche Bestandteile lassen sich beim Absaugen über Kieselgur/Kieselgel entfernen. Die organische Phase wird mit Wasser nachgewaschen, mit Natriumsulfat getrocknet und eingedampft. Das Rohprodukt wird durch Säulenchromatographie (Kieselgel 60, Merck, 40-63 µm, Essigester:Toluol = 4:1) gereinigt`
Ausbeute: 4,97 g (10,2 mmol)
DC (Essigester:Toluol = 4:1)
$R_f$ = 0,46

Analog Beispiel XXIV wurden die in Tabelle 3 aufgeführten Verbindungen hergestellt:

**<u>Tabelle 3</u>**

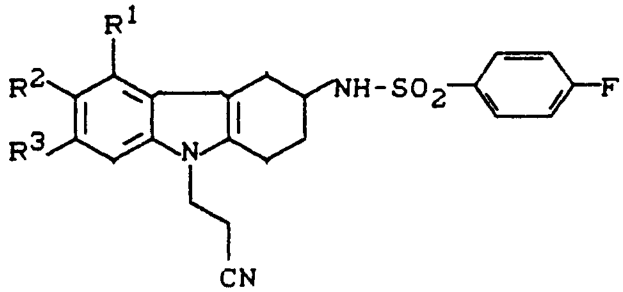

| Bsp.-Nr. | $R^1$ | $R^2$ | $R^3$ | $R_f$ |
|---|---|---|---|---|
| XXV | H | $-CH_2-$ 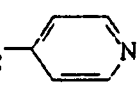 | H | 0,36 a) |
| XXVI | H | $-CH_2-$ 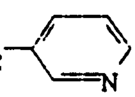 | H | 0,46 a) |
| XXVII | H | H | $-CH_2-$ 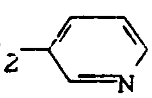 | 0,27 a) |
| XXVIII | H | $-(CH_2)_2-$ 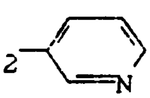 | H | 0,40 a) |
| XXIX | H | $-CH_2-$ 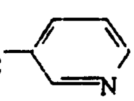 | H | 0,34 b) |
| XXX | 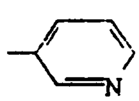 | H | H | 0,48 a) |
| XXXI | H | H | 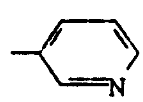 | 0,24 a) |

a) Essigester : Toluol = 4:1
b) Essigester : Toluol = 2:1

Herstellbeispiele
(Verbindungen der allgemeinen Formel I)

Beispiel 1

3-(4-Chlorphenylsulfonamido)-6-fluor-9-(2-(5-tetrazolyl)ethyl)-1,2,3,4-tetrahydrocarbazol

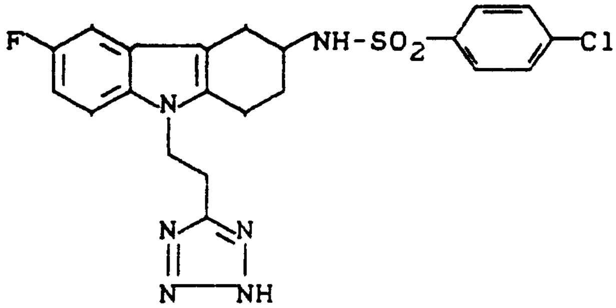

2,9 g (6,7 mmol) der Verbindung aus Beispiel XV werden mit 2,18 g (33,6 mmol) Natriumazid und 4,62 g (33,6 mmol) Triethylammoniumchlorid in 30 ml abs. Dimethylformamid unter Schutzgas 17 Stunden unter Rückfluß umgesetzt. Man verdünnt mit 1 M Schwefelsäure und Essigester, trennt die Phasen und wäscht die organische Phase mehrfach mit Schwefelsäure. Nach der Natriumsulfattrocknung dampft man das Lösemittel ab und reinigt chromatographisch (Kieselgel 60, Merck, 40-63 µm, Dichlormethan : Methanol = 10:1).
Ausbeute: 2,1 g (4,4 mmol)
$R_f$ = 0,13 (Dichlormethan : Methanol = 10:1)

Beispiel 2

3-(4-Fluorphenylsulfonamido)-9-(2-methoxycarbonyl-ethyl)-6-(2-pyridyl-methoxy)-1,2,3,4-tetrahydrocarbazol

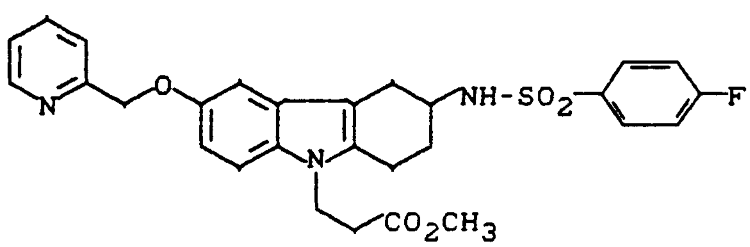

1,5 g (3,34 mmol) 3-(4-Fluorphenylsulfonamido)-6-hydroxy-9-(2-methoxycarbonyl-ethyl)-1,2,3,4-tetrahydrocarbazol werden bei Raumtemperatur unter Stickstoff in 30 ml Dimethylformamid p.a. gelöst und mit 303 mg (10,08 mmol) Natriumhydrid (80%ig, stabilisiert mit Paraffinöl) umgesetzt. Nach beendeter Wasserstoffentwicklung setzt man 0,55 g (3,34 mmol) 2-Chlormethylpyridin-Hydrochlorid zu und rührt 1 Stunde bei 60°C. Die erhaltene Reaktionsmischung wird mit Wasser versetzt und mehrmals mit Essigester extrahiert. Die organischen Extrakte werden mit Natriumsulfat getrocknet und eingedampft. Zur Trennung der Produkte chromatographiert man an Kieselgel 60 (Merck, 40-63 µm, Toluol : Essigester = 4:1, bis 2:1),

Ausbeuten: 120 mg (0,22 mmol) Titelprodukt mit
$R_f$ = 0,41 (Toluol : Essigester 1:1)
97 mg (0,18 mmol) 6-Hydroxy-9-(2-methoxycarbonyl-ethyl)-3-(N-(2-pyridylmethyl)-N-(4-fluorphenyl)-sulfonamido)-1,2,3,4-tetrahydrocarbazol mit $R_f$ = 0,35 (Toluol:Essigester 1:1)
189 mg (0,30 mmol) 9-(2-Methoxycarbonyl-ethyl)-6-(2-pyridyl-methoxy)-3-(N-(2-pyridyl-

methyl)-N-(4-fluorphenyl)sulfonamido)-1,2,3,4-tetrahydrocarbazol mit $R_f$ = 0,20 (Toluol : Essigester 1:1)

Analog der Vorschrift für Beispiel 2 wurden die in Tabelle 4 aufgeführten Verbindungen hergestellt:

**Tabelle 4**

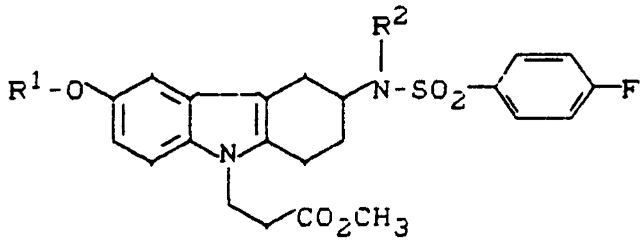

| Bsp.-Nr. | $R^1$ | $R^2$ | $R_f$ *) |
|---|---|---|---|
| 3 | $-CH_2-$ 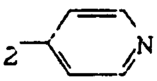 | H | 0,24 |
| 4 | H | $-CH_2-$ 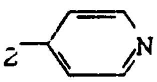 | 0,13 |
| 5 | $-CH_2-$ 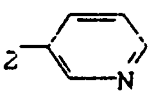 | H | 0,26 |
| 6 | H | $-CH_2-$ 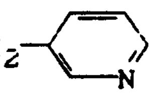 | 0,15 |

*) Toluol:Essigester = 1:1

Beispiel 7 und Beispiel 8

(3R)-(4-Fluorphenylsulfonamido)-9-(2-methoxycarbonylethyl)-6-(2-(3-pyridyl)ethyl-carbonyl)-1,2,3,4-tetrahydrocarbazol (7) und (3R)-(4-Fluorphenylsulfonamido)-9-(2-methoxycarbonyl-ethyl)-8-(2-(3-pyridyl)-ethyl-carbonyl)-1,2,3,4-tetrahydrocarbazol (8)

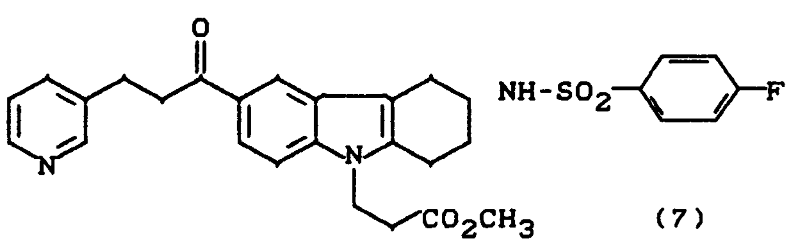

(7)

und

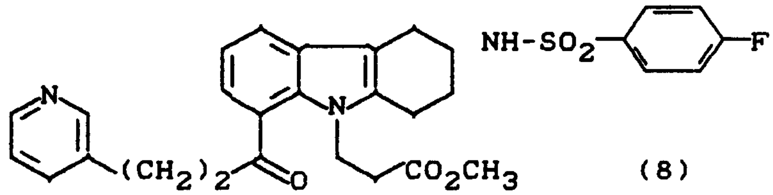

(8)

3,10 g (7,2 mmol) der Verbindung aus Beispiel XI werden in 60 ml 1,2-Dichlorethan p.a. gelöst, mit 2,23 g (10,8 mmol) 3-Pyridylpropionsäurechlorid-Hydrochlorid und 1,92 g (14,2 mmol) Aluminiumchlorid (wasserfrei) versetzt und 2 Tage unter Rückfluß gekocht. Man gießt die erkaltete Reaktionsmischung auf 100 ml gesättigte wäßrige Natriumhydrogencarbonatlösung und extrahiert mehrfach mit Dichlormethan; die organische Phase wird mit Natriumsulfat getrocknet und eingeengt. Zur Trennung der Isomeren chromatographiert man an Kieselgel 60 (Merck, 40-63 µm, Toluol:Essigester = 2:1).

| | |
|---|---|
| Ausbeute: | an (22) 0,52 g (0,9 mmol) und |
| | an (23) 0,41 g (0,7 mmol); |
| | die angefallene Mischfraktion läßt sich weiter auftrennen, |
| DC (Toluol : Essigester = 1:1) | $R_f$ = 0,16 (22) |
| | $R_f$ = 0,12 (23) |

Beispiel 9

(3R)-(4-Fluorphenylsulfonamido)-9-(2-methoxycarbonylethyl)-6-(3-(3-pyridyl)-propyl)-1,2,3,4-tetrahydrocarbazol

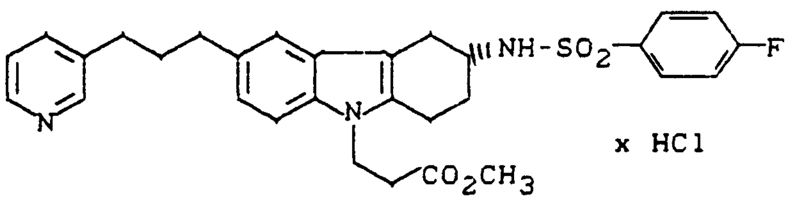

260 mg (0,46 mmol) der Verbindung aus Beispiel (7) werden in 25 ml Tetrahydrofuran abs. bei Raumtemperatur mit 17,4 mg (0,46 mmol) Natriumboranat umgesetzt. Wenn nach 4 Stunden noch Ausgangsmaterial vorliegt, gibt man weitere 17,4 mg (0,46 mmol) Natriumboranat zu und rührt über Nacht. Man gibt darauf 30 ml 0,1 M Salzsäure zu und extrahiert mehrmals mit je 25 ml Essigester. Die mit Natriumsulfat getrockneten organischen Phasen werden eingedampft und das anfallende Rohprodukt chromatographisch gereinigt (Kieselgel 60, Merck, 40-63 µm, Toluol : Essigester = 4:1 bis 2:1),
Ausbeute: 127 mg (0,23 mmol)
DC (Toluol : Essigester = 1:4) $R_f$ = 0,66

Beispiel 10

(3R)-((4-Fluorphenylsulfonamido)-9-(2-methoxycarbonylethyl)-8-(3-(3-pyridyl)-propyl-1,2,3,4-tetrahydrocarbazol

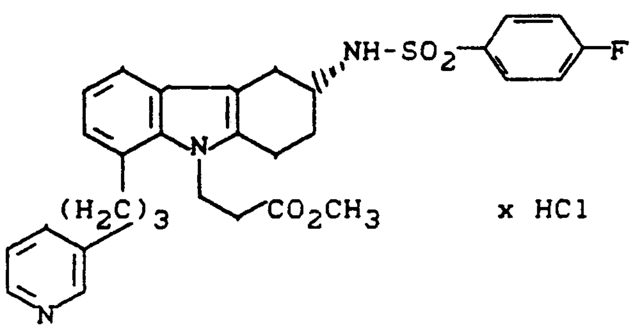

Die Titelverbindung wird in Analogie zur Vorschrift des Beispiels 9 hergestellt.
DC: $R_f$ = 0,59 (Toluol : Essigester = 1:4)

Beispiel 11

9-(2-Carboxyethyl)-3-(4-fluorphenylsulfonamido)-6-(3-pyridyl-methyl)-1,2,3,4-tetrahydro-carbazol-Hydrochlorid

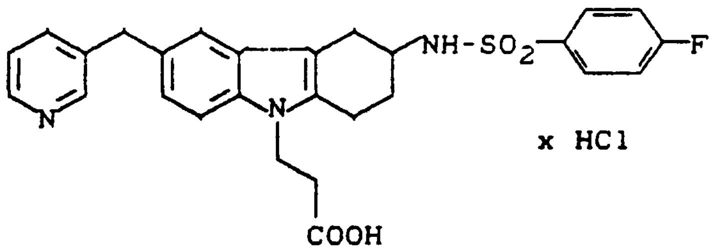

x HCl

4,74 g (9,7 mmol) der Verbindung aus Beispiel XXVI werden in 20 ml Ethanol gelöst, mit 17 ml 2 M NaOH und 20 ml Wasser versetzt und 2 Stunden unter Rückfluß gekocht. Man extrahiert die abgekühlte Reaktionsmischung einmal mit Dichlormethan und einmal mit Ether, stellt die wäßrige Phase mit 2 M Salzsäure auf pH 5, saugt den entstandenen Niederschlag ab und wäscht mit Wasser nach. Das Produkt wird im Vakuum über $P_4O_{10}$ getrocknet.
Ausbeute: 5,03 g (9,2 mmol)
DC ($CH_2Cl_2$ : $CH_3OH$ = 10:1) $R_f$ = 0,30

In Analogie zur Vorschrift des Beispiels 11 lassen sich die in den Tabelle 5 und 6 aufgeführten Verbindungen herstellen.

**Tabelle 5**

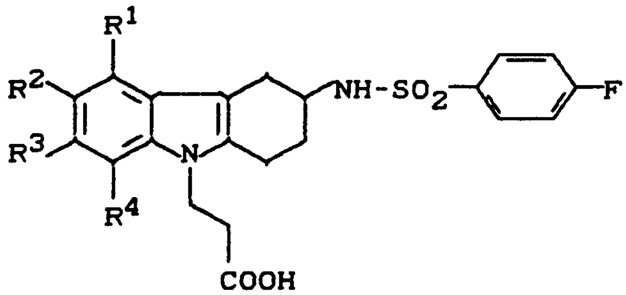

| Bsp.-Nr. | $R^1$ | $R^2$ | $R^3$ | $R^4$ | $R_f$ |
|---|---|---|---|---|---|
| 12 | H | $-O-CH_2-$ 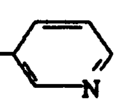 | H | H | 0,25 |
| 13 | H | $-O-CH_2-$ 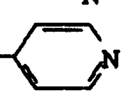 | H | H | 0,16 |

Fortsetzung Tabelle 5

| Bsp.-Nr. | $R^1$ | $R^2$ | $R^3$ | $R^4$ | $R_f$ |
|---|---|---|---|---|---|
| 14 | H | $-O-CH_2-$ 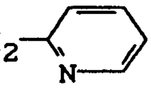 | H | H | 0,35 |
| 15 | H | $-CH_2-$ 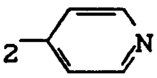 | H | H | 0,12 |
| 16 | H | H | $-CH_2-$ 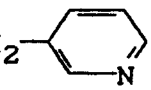 | H | 0,27 |
| 17 | H | $-(CH_2)_2-$ 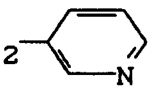 | H | H | 0,31 |
| 18 | $-CH_2-$ 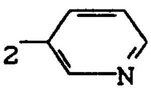 | H | H | H | 0,35 |
| 19 | H | H | H | $-CO-(CH_2)_2-$ 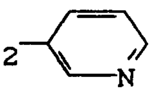 | 0,05 |
| 20 | H | $-CO-(CH_2)_2-$ 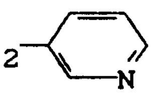 | H | H | 0,05 |
| 21 | H | $-(CH_2)_3-$ 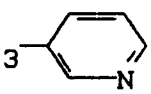 | H | H | 0,17 |
| 22 | H | H | H | $-(CH_2)_3-$ 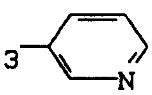 | 0,10 |
| 23 | 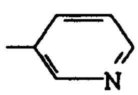 | H | H | H | 0,29 |
| 24 | H | H | 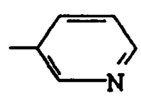 | H | 0,19 |

*) Laufmittel= $CH_2Cl_2$ : $CH_3OH$ = 10:1

Tabelle 6

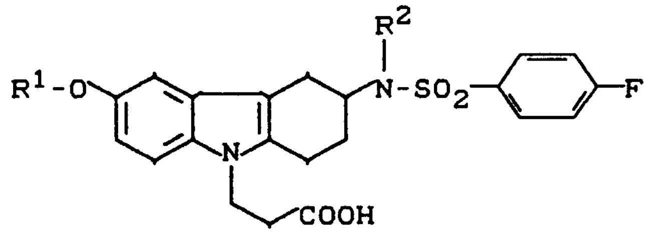

| Bsp.-Nr. | $R^1$ | $R^2$ | $R_f$*) |
|---|---|---|---|
| 25 | 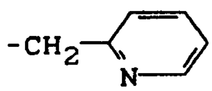 | 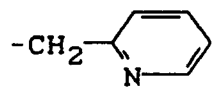 | 0,50 |
| 26 | H | 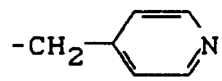 | 0,24 |

*) Laufmittel = Dichlormethan : Methanol 10:1

## Patentansprüche

**1.** Heterocyclisch substituierte Cycloalkano[b]indolsulfonamide der allgemeinen Formel (I)

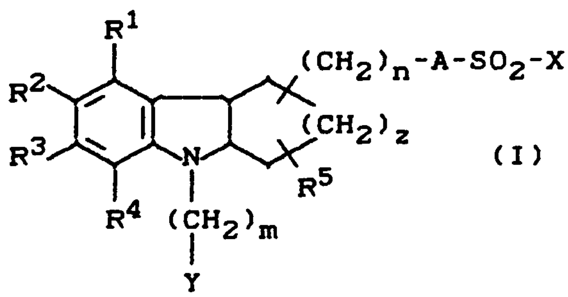

(I)

in welcher

$R^1$, $R^2$, $R^3$, $R^4$ und $R^5$ gleich oder verschieden sind und

- für Wasserstoff, Nitro, Cyano, Halogen, Trifluormethyl, Carboxy, Hydroxy oder Trifluormethoxy stehen, oder
- für eine Gruppe der Formel $-S(O)_wR^6$ stehen,

worin

$R^6$ - geradkettiges oder verzweigtes Alkyl mit bis zu 8 Kohlenstoffatomen oder Aryl mit 6 bis 10 Kohlenstoffatomen bedeutet, das seinerseits durch Halogen, Nitro, Cyano oder Trifluormethyl substituiert ist

und

w - eine Zahl 0, 1 oder 2 bedeutet,

oder

- für geradkettiges oder verzweigtes Alkoxy oder Alkoxycarbonyl mit jeweils bis zu 10 Kohlenstoffatomen oder Benzyloxy stehen, oder
- für eine Gruppe der Formel $-NR^7R^8$ stehen,

worin

$R^7$ und $R^8$ gleich oder verschieden sind und
- Wasserstoff, geradkettiges oder verzweigtes Alkyl oder Acyl mit jeweils bis zu 8 Kohlenstoffatomen oder
- Aryl mit 6 bis 10 Kohlenstoffatomen bedeuten,

oder
- für Cycloalkyl mit 3 bis 8 Kohlenstoffatomen oder
- für Aryl mit 6 bis 10 Kohlenstoffatomen stehen, oder
- für geradkettiges oder verzweigtes Alkyl oder Alkenyl mit jeweils bis zu 10 Kohlenstoffatomen stehen, die gegebenenfalls durch Halogen, Hydroxy, Carboxy, Cyano, Aryl mit 6 bis 10 Kohlenstoffatomen, geradkettiges oder verzweigtes Alkyl, Alkoxy oder Alkoxycarbonyl mit jeweils bis zu 8 Kohlenstoffatomen oder durch eine Gruppe der Formel

$-S(O)_wR^6$ oder

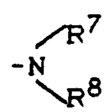

substituiert sind,

worin

w, $R^6$, $R^7$ und $R^8$ die oben angegebene Bedeutung haben,

oder
- für eine Gruppe der Formel

-D, -E-O-L-D, -E-NH-L-D, -E-CO-L-D oder -E-D

stehen,

worin

D - einen 3- bis 7-gliedrigen, gesättigten oder ungesättigten Heterocyclus mit bis zu 4 Heteroatomen aus der Reihe N, O oder S bedeutet, an den weitere aromatische oder heterocyclische Ringe ankondensiert sein können,

und

E und L gleich oder verschieden sind und
- eine direkte Bindung oder
- geradkettiges oder verzweigtes Alkylen oder Alkenylen mit bis zu 10 Kohlenstoffatomen bedeuten,

X

- für Aryl mit 6 bis 10 Kohlenstoffatomen steht, das gegebenenfalls bis zu 5-fach gleich oder verschieden durch Nitro, Halogen, Cyano, Trifluormethyl, Trifluormethoxy, Trifluormethylthio, Hydroxy, Carboxy, Aryl oder Aryloxy mit 6 bis 10 Kohlenstoffatomen, geradkettiges oder verzweigtes Alkoxy oder Alkoxycarbonyl mit jeweils bis zu 8 Kohlenstoffatomen oder durch geradkettiges oder verzweigtes Alkyl mit bis zu 8 Kohlenstoffatomen substituiert ist, wobei das Alkyl seinerseits durch Carboxy, Hydroxy, Alkoxycarbonyl mit bis zu 6 Kohlenstoffatomen, durch Aryl mit 6 bis 10 Kohlenstoffatomen,
  oder
  durch eine Gruppe der Formel $-S(O)_wR^6$ oder $-NR^7R^8$ substituiert sein kann,

  worin

  w, $R^6$, $R^7$ und $R^8$ die oben angegebene Bedeutung haben,

  oder
- für geradkettiges oder verzweigtes Alkyl mit bis zu 8 Kohlenstoffatomen oder
- für Trifluormethyl steht,

m - für die Zahl 1, 2, 3 oder 4 steht,

n - für die Zahl 0, 1 oder 2 steht,

z - für eine Zahl 1, 2, 3 oder 4 steht,

A - für eine direkte Bindung oder für eine Gruppe der Formel

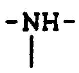

oder -N-E-D steht,

worin

D und E die oben angegebene Bedeutung haben,

Y

- für die Gruppe der Formel -CO-G steht,

  worin

  G - Hydroxy, Alkoxy mit bis zu 8 Kohlenstoffatomen, Phenoxy oder eine Gruppe der Formel $-NR^7R^8$ oder $-NH-SO_2-R^6$ bedeutet,

  worin

  $R^6$, $R^7$ und $R^8$ die oben angegebene Bedeutung haben,

  oder
- für Tetrazolyl steht,

mit der Maßgabe, daß entweder der Substituent A für den Rest -N-E-D oder mindestens einer der Substituenten $R^1$, $R^2$, $R^3$, $R^4$ und $R^5$ für eine der Gruppen -D, -E-O-L-D, -E-NH-L-D, -E-CO-L-D oder -E-D stehen müssen,

gegebenenfalls in einer isomeren Form und ihre Salze.

**2.** Heterocyclisch substituierte Cycloalkano[b]indolsulfonamide der Formel nach Anspruch 1, in welcher

$R^1$, $R^2$, $R^3$, $R^4$ und $R^5$ gleich oder verschieden sind und

- für Wasserstoff, Fluor, Chlor, Brom, Nitro, Cyano, Trifluormethyl,

Carboxy, Hydroxy oder Trifluormethoxy stehen,

- für eine Gruppe der Formel $-S(O)_wR^6$ stehen,

worin

$R^6$ - geradkettiges oder verzweigtes Alkyl mit bis zu 6 Kohlenstoffatomen oder Phenyl bedeutet, das seinerseits durch Fluor, Chlor, Brom, Nitro, Cyano oder Trifluormethyl substituiert sein kann,

w - eine Zahl 0, 1 oder 2 bedeutet,

oder

- für geradkettiges oder verzweigtes Alkoxy oder Alkoxycarbonyl mit jeweils bis zu 8 Kohlenstoffatomen stehen, oder
- für eine Gruppe der Formel $-NR^7R^8$ stehen,

worin

$R^7$ und $R^8$ gleich oder verschieden sind und

- Wasserstoff, geradkettiges oder verzweigtes Alkyl mit bis zu 6 Kohlenstoffatomen oder Phenyl bedeuten,

oder

- für Cyclopropyl, Cyclopentyl, Cyclohexyl oder Phenyl stehen, oder
- für geradkettiges oder verzweigtes Alkyl oder Alkenyl mit jeweils bis zu 8 Kohlenstoffatomen stehen, die gegebenenfalls durch Fluor, Chlor, Brom, Hydroxy, Carboxy, Cyano, Phenyl, geradkettiges oder verzweigtes Alkyl, Alkoxy oder Alkoxycarbonyl mit jeweils bis zu 6 Kohlenstoffatomen oder durch eine Gruppe der Formel $-S(O)_wR^6$ oder $-NR^7R^8$ substituiert sind,

worin

w, $R^6$, $R^7$ und $R^8$ die oben angegebene Bedeutung haben,

oder

- für einen Rest oder eine Gruppe der Formel

-D, -E-O-L-D, -E-NH-L-D, -E-CO-L-D oder -E-D stehen,

worin

D - Pyridyl, Chinolyl, Tetrazolyl, Benzthiazolyl, Isochinolyl, Benzimidazolyl, Pyrimidyl, Pyrrolyl, Thienyl, Furyl, Imidazolyl oder Thiazolyl bedeutet,
und

E und L gleich oder verschieden sind und

- eine direkte Bindung oder
- geradkettiges oder verzweigtes Alkyl oder Alkenyl mit jeweils bis zu 8 Kohlenstoffatomen bedeuten,

X

- für Phenyl steht, das gegebenenfalls bis zu 4-fach gleich oder verschieden durch Nitro, Hydroxy, Fluor, Chlor, Brom, Jod, Cyano, Trifluormethyl, Trifluormethoxy, Hydroxy, Carboxy, Phenyl, Phenoxy, geradkettiges oder verzweigtes Alkoxy oder Alkoxycarbonyl mit jeweils bis zu 6 Kohlenstoffatomen oder durch geradkettiges oder verzweigtes Alkyl mit bis zu 6 Kohlenstoffatomen substituiert ist, oder

- für geradkettiges oder verzweigtes Alkyl mit bis zu 6 Kohlenstoffatomen oder für Trifluormethyl steht,

m - für die Zahl 1, 2 oder 3 steht,

n - für die Zahl 0 oder 1 steht,

z - für eine Zahl 1, 2 oder 3 steht,

A - für eine direkte Bindung oder für eine Gruppe der Formel

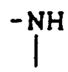

oder

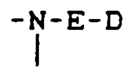

steht,

worin

D und E die oben angegebene Bedeutung haben,

Y

- für die Gruppe der Formel -CO-G setht,

  worin

  G - Hydroxy, Alkoxy mit bis zu 6 Kohlenstoffatomen, Phenoxy oder die Gruppe $-NHSO_2R^6$ bedeutet,

  worin

  $R^6$ die oben angegebene Bedeutung hat,

  oder
- für Tetrazolyl steht,

mit der Maßgabe, daß entweder der Substituent A für den Rest -N-E-D oder mindestens einer der Substituenten $R^1$, $R^2$, $R^3$, $R^4$ und $R^5$ für eine der Gruppen -D, -E-O-L-D, -E-NH-L-D, -E-CO-L-D oder -E-D stehen müssen,

gegebenenfalls in einer isomeren Form und ihre Salze.

**3.** Heterocyclisch substituierte Cycloalkano[b]indolsulfonamide der Formel nach Anspruch 1, in welcher

$R^1$, $R^2$, $R^3$, $R^4$ und $R^5$ gleich oder verschieden sind und

- für Wasserstoff, Fluor oder Chlor stehen,
- für eine Gruppe der Formel $-S(O)_w-R^6$ stehen,

  worin

  $R^6$ - geradkettiges oder verzweigtes Alkyl mit bis zu 4 Kohlenstoffatomen oder Phenyl bedeutet, das seinerseits durch Fluor, Chlor oder Brom substituiert sein kann

  und

  w - die Zahl 2 bedeutet,

oder

- für geradkettiges oder verzweigtes Alkoxy oder Alkoxycarbonyl mit jeweils bis zu 4 Kohlenstoffatomen stehen, oder
- für die Gruppe der Formel $-NR^7R^8$ stehen,

worin

$R^7$ und $R^8$ gleich oder verschieden sind und

- Wasserstoff, geradkettiges oder verzweigtes Alkyl mit bis zu 4 Kohlenstoffatomen oder Phenyl bedeuten,

oder

- für geradkettiges oder verzweigtes Alkyl mit bis zu 6 Kohlenstoffatomen stehen, die gegebenenfalls durch Fluor, Chlor, Brom, Hydroxy, Cyano oder Phenyl substituiert sind,

oder

- für einen Rest oder eine Gruppe der Formel -D, -E-O-L-D, -E-CO-L-D oder -E-D stehen,

worin

D - Pyridyl, Pyrimidyl, Imidazolyl oder Thiazolyl bedeutet,

und

E und L gleich oder verschieden sind und

- eine direkte Bindung oder
- geradkettiges oder verzweigtes Alkyl mit bis zu 6 Kohlenstoffatomen bedeuten,

X - für Phenyl steht, das gegebenenfalls bis zu 3-fach gleich oder verschieden durch Fluor, Chlor, Brom, Cyano, Trifluormethyl, Trifluormethoxy oder durch geradkettiges oder verzweigtes Alkyl, Alkoxy oder Alkoxycarbonyl mit jeweils bis zu 4 Kohlenstoffatomen substituiert ist,

m - für die Zahl 1 oder 2 steht,

n - für die Zahl 0 oder 1 steht,

z - für eine Zahl 1 oder 2 steht,

A - für eine direkte Bindung oder für eine Gruppe der Formel

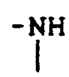

oder

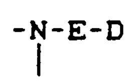

steht,

worin

D und E die oben angegebene Bedeutung haben,

Y

- für die Gruppe der Formel -CO-G steht,

worin

G - Hydroxy, Alkoxy mit bis zu 4 Kohlenstoffatomen oder die Gruppe $-NHSO_2R^6$ bedeutet,

worin

$R^6$ die oben angegebene Bedeutung hat,
oder
- für Tetrazolyl steht

mit der Maßgabe, daß entweder der Substituent A für den Rest

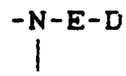

oder mindestens einer der Substituenten $R^1$, $R^2$, $R^3$, $R^4$ und $R^5$ für eine der Gruppen -D, -E-O-L-D, -E-NH-L-D, -E-CO-L-D oder -E-D stehen müssen,

gegebenenfalls in einer isomeren Form und ihre Salze.

**4.** Heterocyclisch substituierte Cycloalkano[b]-indolsulfonamide nach Anspruch 1 zur Bekämpfung von Krankheiten.

**5.** Verfahren zur Herstellung von heterocyclisch substituierten Cycloalkano[b]-indolsulfonamiden der allgemeinen Formel

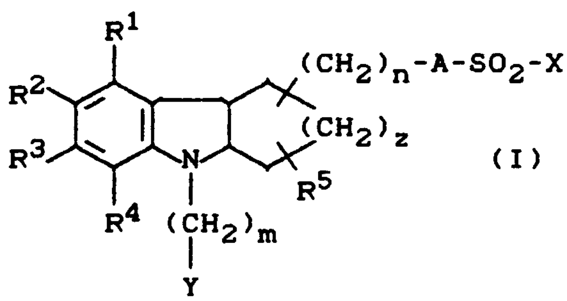

(I)

in welcher

$R^1$, $R^2$, $R^3$, $R^4$ und $R^5$ gleich oder verschieden sind und
- für Wasserstoff, Nitro, Cyano, Halogen, Trifluormethyl, Carboxy, Hydroxy oder Trifluormethoxy stehen, oder
- für eine Gruppe der Formel $-S(O)_wR^6$ stehen,

worin

$R^6$ - geradkettiges oder verzweigtes Alkyl mit bis zu 8 Kohlenstoffatomen oder Aryl mit 6 bis 10 Kohlenstoffatomen bedeutet, das seinerseits durch Halogen, Nitro, Cyano oder Trifluormethyl substituiert ist

und

w - eine Zahl 0, 1 oder 2 bedeutet,

oder

- für geradkettiges oder verzweigtes Alkoxy oder Alkoxycarbonyl mit jeweils bis zu 10 Kohlenstoffatomen oder Benzyloxy stehen, oder
- für eine Gruppe der Formel $-NR^7R^8$ stehen,

worin

$R^7$ und $R^8$ gleich oder verschieden sind und
  - Wasserstoff, geradkettiges oder verzweigtes Alkyl oder Acyl mit jeweils bis zu 8 Kohlenstoffatomen oder
  - Aryl mit 6 bis 10 Kohlenstoffatomen bedeuten,

oder
- für Cycloalkyl mit 3 bis 8 Kohlenstoffatomen oder
- für Aryl mit 6 bis 10 Kohlenstoffatomen stehen, oder
- für geradkettiges oder verzweigtes Alkyl oder Alkenyl mit jeweils bis zu 10 Kohlenstoffatomen stehen, die gegebenenfalls durch Halogen, Hydroxy, Carboxy, Cyano, Aryl mit 6 bis 10 Kohlenstoffatomen, geradkettiges oder verzweigtes Alkyl, Alkoxy oder Alkoxycarbonyl mit jeweils bis zu 8 Kohlenstoffatomen oder durch eine Gruppe der Formel

$-S(O)_wR^6$ oder

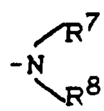

substituiert sind,

worin

w, $R^6$, $R^7$ und $R^8$ die oben angegebene Bedeutung haben,

oder
- für eine Gruppe der Formel
  -D, -E-O-L-D, -E-NH-L-D, -E-CO-L-D oder -E-D

stehen,

worin

D - einen 3- bis 7-gliedrigen, gesättigten oder ungesättigten Heterocyclus mit bis zu 4 Heteroatomen aus der Reihe N, O oder S bedeutet, an den weitere aromatische oder heterocyclische Ringe ankondensiert sein können,

und

E und L gleich oder verschieden sind und
  - eine direkte Bindung oder
  - geradkettiges oder verzweigtes Alkylen oder Alkenylen mit bis zu 10 Kohlenstoffatomen bedeuten,

X

- für Aryl mit 6 bis 10 Kohlenstoffatomen steht, das gegebenenfalls bis zu 5-fach gleich oder verschieden durch Nitro, Halogen, Cyano, Trifluormethyl, Trifluormethoxy, Trifluormethylthio, Hydroxy, Carboxy, Aryl oder Aryloxy mit 6 bis 10 Kohlenstoffatomen, geradkettiges oder

verzweigtes Alkoxy oder Alkoxycarbonyl mit jeweils bis zu 8 Kohlenstoffatomen oder durch geradkettiges oder verzweigtes Alkyl mit bis zu 8 Kohlenstoffatomen substituiert ist, wobei das Alkyl seinerseits durch Carboxy, Hydroxy, Alkoxycarbonyl mit bis zu 6 Kohlenstoffatomen, durch Aryl mit 6 bis 10 Kohlenstoffatomen,
oder
durch eine Gruppe der Formel $-S(O)_wR^6$ oder $-NR^7R^8$ substituiert sein kann,

worin

w, $R^6$, $R^7$ und $R^8$ die oben angegebene Bedeutung haben,

oder
- für geradkettiges oder verzweigtes Alkyl mit bis zu 8 Kohlenstoffatomen oder
- für Trifluormethyl steht,

m - für die Zahl 1, 2, 3 oder 4 steht,
n - für die Zahl 0, 1 oder 2 steht,
z - für eine Zahl 1, 2, 3 oder 4 steht,
A - für eine direkte Bindung oder für eine Gruppe der Formel

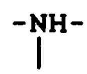

oder

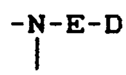

steht,

worin

D und E die oben angegebene Bedeutung haben,

Y
- für die Gruppe der Formel -CO-G steht,

worin

G - Hydroxy, Alkoxy mit bis zu 8 Kohlenstoffatomen, Phenoxy oder eine Gruppe der Formel $-NR^7R^8$ oder $-NH-SO_2-R^6$ bedeutet,

worin

$R^6$, $R^7$ und $R^8$ die oben angegebene Bedeutung haben,

oder
- für Tetrazolyl steht,

mit der Maßgabe, daß entweder der Substituent A für den Rest -N-E-D oder mindestens einer der Substituenten $R^1$, $R^2$, $R^3$, $R^4$ und $R^5$ für eine der Gruppen -D, -E-O-L-D, -E-NH-L-D, -E-CO-L-D oder -E-D stehen müssen,

gegebenenfalls in einer isomeren Form und ihre Salze, dadurch gekennzeichnet, daß man
[A] im Fall, daß m für die Zahl 2 steht,

Verbindungen der allgemeinen Formel (VIII)

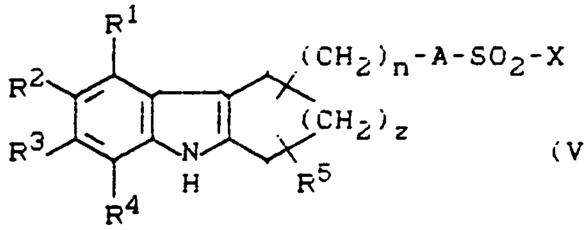

(VIII)

in welcher

$R^1$, $R^2$, $R^3$, $R^4$, $R^5$, z, n, A und X die oben angegebene Bedeutung haben,

mit Acrylnitril, gegebenenfalls in Anwesenheit einer Base in inerten Lösemitteln zu Verbindungen der allgemeinen Formel (IX)

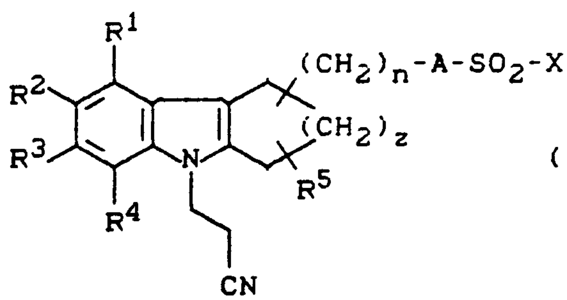

(IX)

in welcher

$R^1$, $R^2$, $R^3$, $R^4$, $R^5$, z, n, A und X die oben angegebene Bedeutung haben,

umsetzt,

im Fall, daß Y für den Tetrazolring steht, diese in Anwesenheit von Triethylaminhydrochlorid mit Natriumazid umsetzt,

oder im Fall der Herstellung der Säuren (Y = COOH) die Cyanogruppe nach üblicher Methode verseift,

im Fall der Herstellung der Ester (Y = $C_1$-$C_8$-Alkoxycarbonyl, Phenoxycarbonyl) die Säuren mit den entsprechenden Alkoholen in Gegenwart eines Katalysators gegebenenfalls in inerten Lösungsmitteln nach üblicher Methode umsetzt,

im Fall der Herstellung der Amide und Acylsulfonsäureamide (Y = $-CONR^7R^8$, $-CO-NHSO_2-R^6$) entweder die Ester oder die Säuren nach üblicher Aktivierung, mit den Aminen oder Sulfonsäureamiden der allgemeinen Formeln (Xa) und (Xb)

$HNR^7R^8$ (Xa)

$NH_2SO_2-R^6$ (Xb)

in welcher

$R^6$, $R^7$ und $R^8$ die oben angegebene Bedeutung haben,

gegebenenfalls in Anwesenheit eines Katalysators umsetzt,

oder indem man
[B] Verbindungen der allgemeinen Formel (XI)

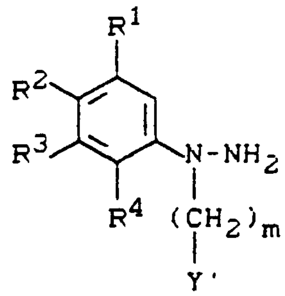

(XI)

in welcher

$R^1$, $R^2$, $R^3$, $R^4$, und m die oben angegebene Bedeutung haben,

und

Y' - für ($C_1$-$C_4$)-Alkoxycarbonyl oder Cyano steht,

mit Cycloalkanonsulfonamiden der allgemeinen Formel (XII)

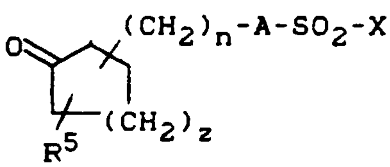

(XII)

in welcher

z, n, A, $R^5$ und X die oben angegebene Bedeutung haben,

in inerten Lösemitteln, gegebenenfalls in Anwesenheit eines Katalysators umsetzt,

im Fall der Herstellung der Säuren (Y = COOH) die Ester oder Cyanogruppe nach üblicher Methode verseift,

im Fall der Herstellung von Estern (Y = $C_1$-$C_8$-Alkoxycarbonyl, Phenoxycarbonyl) die Säuren mit den entsprechenden Alkoholen in Gegenwart eines Katalysators gegebenenfalls in inerten Lösemitteln nach üblicher Methode umsetzt, oder Ester nach üblichen Methoden umestert,

im Fall der Herstellung der Amide und Acylsulfonsäureamide (Y = $-CONR^7R^8$, $-CO-NHSO_2-R^6$) entweder direkt die Ester oder die Säuren nach üblicher Aktivierung, mit den Aminen oder Sulfonsäureamiden der allgemeinen Formeln (Xa) und (Xb) gegebenenfalls in Anwesenheit eines Katalysators umsetzt,

im Fall, daß Y für den Tetrazolring steht, die Cyanoverbindung (Y = CN) in Anwesenheit von Triethylaminhydrochlorid mit Natriumazid umsetzt,

oder indem man
[C] in Verbindungen der allgemeinen Formel (Ia)

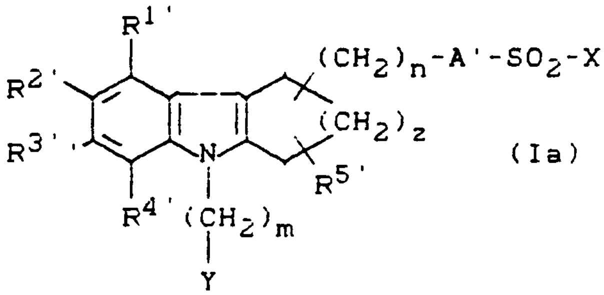

(Ia)

in welcher

$R^{1'}$, $R^{2'}$, $R^{3'}$, $R^{4'}$ und $R^{5'}$ die angegebene Bedeutung haben, jedoch nicht für eine Gruppe der Formel -D, -E-O-L-D, -E-NH-L-D, -E-CO-L-D oder -ED steht,

A' für die Gruppe -NH- steht und

m, Y, X und z die angegebene Bedeutung haben,

die Reste der Formel -D, -E-O-L-D, -E-NH-L-D, -E-CO-L-D oder E-D nach üblichen Methoden, beispielsweise durch nucleophile oder elektrophile aromatische Substitution einführt, und gegebenenfalls Carbonylgruppen nach üblichen Methoden zu Methylengruppen reduziert,

im Falle der Herstellung der Säuren (Y = -COOH) die Ester nach üblicher Methode verseift,

im Fall der Variation der Ester (Y = $C_1$-$C_8$-Alkoxycarbonyl, Phenoxycarbonyl) die Säuren mit den entsprechenden Alkoholen in Gegenwart eines Katalysators gegebenenfalls in inerten Lösemitteln nach üblicher Methode umsetzt,

im Fall der Amide und Acylsulfonsäureamide (Y = $-CONR^7R^8$, $-CO-NHSO_2-R^6$) entweder direkt die Ester oder die Säuren nach üblicher Aktivierung, mit den Aminen oder Sulfonsäureamiden der allgemeinen Formeln (Xa) und (Xb) gegebenenfalls in Gegenwart eines Katalysators umsetzt,

und dann im Fall der heterocyclisch substituierten Cycloalkano[b]-dihydroindolsulfonamide die nach Verfahren [A], [B] oder [C] hergestellten heterocyclisch substituierten Indolsulfonamide in Gegenwart eines Reduktionsmittels in inerten Lösemitteln reduziert,

gegebenenfalls die Isomeren trennt und

im Fall der Herstellung der Salze mit einer entsprechenden Base oder Säure umsetzt.

**6.** Arzneimittel enthaltend mindestens ein heterocyclisch substituiertes Cycloalkano[b]-indolsulfonamid nach Anspruch 1.

**7.** Verfahren zur Herstellung eines Arzneimittels nach Anspruch 6, dadurch gekenzeichnet, daß man Verbindungen der allgemeinen Formel (I) gegebenenfalls mit üblichen Hilfs- und Trägerstoffen in eine geeignete Applikationsform bringt.

**8.** Verwendung von heterocyclisch substituierten Indolsulfonamiden nach Anspruch 1 zur Herstellung von Arzneimitteln.

**9.** Verwendung von heterocyclisch substituierten Cycloalkano[b]-indolsulfonamiden nach Anspruch 1 zur Herstellung von Arzneimitteln zur Behandlung von thromboembolischen Erkrankungen, Ischämien, Arteriosklerose, Asthma, Allergien sowie zur Prophylaxe des Myocardinfarkts.

**10.** Verwendung von heterocyclisch substituierten Cycloalkano[b]-indolsulfonamiden nach Anspruch 1 zur

Bekämpfung von Krankheiten.

Europäisches Patentamt

**EUROPÄISCHER TEILRECHERCHENBERICHT,** der nach Regel 45 des Europäischen Patent-übereinkommens für das weitere Verfahren als europäischer Recherchenbericht gilt

Nummer der Anmeldung

EP 91113731.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| D,X | DE - A1 - 3 631 824 (BAYER) * Ansprüche 1,18,19 * | 1,6-8 | C 07 D 209/94<br>C 07 D 403/06<br>C 07 D 209/80<br>C 07 D 209/86<br>A 61 K 31/405<br>A 61 K 31/41 |
| P,X | US - A - 4 988 820 (BÖSHAGEN) * Anspruch 1; Spalten 12-13, Zeile 47 - Spalte 21, Zeilen 36-57 * | 1,5-8 | |
| A | EP - A2 - 0 300 675 (MERCK) * Verbindungen 2-4; Anspruch 1 * | 5-8 | |
| A | EP - A2 - 0 269 452 (GLAXO) * Anspruch 1 * | 5-8 | |
| A | CHEMICAL ABSTRACTS, Band 69, Nr. 9, 26. August 1968, Columbus, Ohio, USA OKAWA, KATSUAKI et al. "Basic dyes for poly- | 1 | |

RECHERCHIERTE SACHGEBIETE (Int. Cl.⁵)

C 07 D 209/00
C 07 D 403/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9
Unvollständig recherchierte Patentansprüche: -
Nicht recherchierte Patentansprüche: 10
Grund für die Beschränkung der Recherche: Art. 52(4) (Verfahren zur therapeutischen Behandlung des menschlichen oder tierischen Körpers)

| Recherchenort | Abschlußdatum der Recherche | Prüfer |
|---|---|---|
| WIEN | 04-11-1991 | HAMMER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

# EUROPÄISCHER TEILRECHERCHENBERICHT

Nummer der Anmeldung
-2-
EP 91113731.3

| EINSCHLÄGIGE DOKUMENTE | | | KLASSIFIKATION DER ANMELDUNG (Int. Cl.5) |
|---|---|---|---|
| Kategorie | Kennzeichnung des Dokuments mit Angabe, soweit erforderlich, der maßgeblichen Teile | betrifft Anspruch | |
| | acrylonitrile fibers" Seite 3485, Spalte 1, Zusammenfassung-Nr. 37 117u & Japan. 68 07,024, 15 Mar 1968 -- | | |
| A | CHEMICAL ABSTRACTS, Band 85, Nr. 13, 27. September 1976, Columbus, Ohio, USA HAHN, WITOLD et al. "Salts of 1-(omega-guanidinoalkyl)indoles" Seite 621, Spalte 2, Zusammenfassung-Nr. 94 224f & Pol. 78,589, 25 Oct 1975 -- | 1 | RECHERCHIERTE SACHGEBIETE (Int. Cl.5) |
| X | CHEMICAL ABSTRACTS, Band 110, Nr. 9, 27. Februar 1989, Columbus, Ohio, USA BOSHAGEN, HORST et al. "Preparation of cycloalkano (1,2-b)indole-substituted arenesulfonamides as blood platelet aggregation inhibitors" Seite 632, Spalte 1, Zusammenfassung-Nr. 75 309f & Faming Zhuanli Shenqing Gongkai Shuominingshu CN 87,100,773, 02 Sep 1987 ---- | 1,6-8 | |